# EUROPÄISCHE PATENTSCHRIFT

(11) **EP 0 809 627 B1**
(45) Veröffentlichungstag und Bekanntmachung des Hinweises auf die Patenterteilung: **12.09.2001**
(21) Anmeldenummer: 96903893.4
(22) Anmeldetag: 19.02.1996
(51) Int. Cl.: C07C 271/22, C07D 303/16, A61K 31/325, A61K 31/335

(54) **NEUE BORNEOLDERIVATE, VERFAHREN ZU IHRER HERSTELLUNG UND IHRE PHARMAZEUTISCHE VERWENDUNG**
NOVEL BORNEOL DERIVATIVES, METHODS OF MANUFACTURING THEM, AND THEIR PHARMACEUTICAL USE
NOUVEAUX DERIVES DE BORNEOL, PROCEDE DE FABRICATION ET UTILISATION EN PHARMACIE

(30) Priorität: 17.02.1995 DE 19506885
(43) Veröffentlichungstag der Anmeldung: 03.12.1997
(73) Patentinhaber: SCHERING AKTIENGESELLSCHAFT, 13342 Berlin (DE)
(72) Erfinder: KLAR, Ulrich, D-13503 Berlin (DE); GRAF, Hermann, D-13465 Berlin (DE); NEEF, Günter, D-10771 Berlin (DE); BLECHERT, Siegfried, D-13503 Berlin (DE)
(86) Internationale Anmeldenummer: DE9600297
(87) Internationale Veröffentlichungsnummer: WO9625392

(56) Entgegenhaltungen:
- EP-A- 0 253 739
- DE-A- 4 416 374

## Beschreibung

Die Erfindung betrifft neue pharmakologisch wirksame Verbindungen, welche die Fähigkeit haben, die Tubulin-Polymerisation bzw. Tubulin-Depolymerisation zu beeinflussen.

Eine Reihe natürlicher Mitosegifte werden als Antitumormittel eingesetzt bzw. befinden sich in der klinischen Prüfung. Es existieren verschiedene Klassen dieser Mitosegifte, die ihre zytotoxische Wirkung entweder durch Inhibition der Polymerisation von Mikrotubuli im Spindelapparat entfalten (z. B. Vinka-Alkaloide, Colchicin) oder dies durch eine GTP-unabhängige Steigerung der Polymerisation des Tubulins und Verhinderung der Depolymerisation von Mikrotubuli erreichen (z. B. Taxol, Taxotere). Mitosegifte haben aufgrund bisher unverstandener physikochemischer Eigenschaften und durch die Besonderheiten neoplastischer Zellen eine gewisse Selektivität für Tumorzellen, es besteht jedoch auch eine nicht unerhebliche Zytotoxizität gegenüber nicht transformierten Zellen.

Vinka-Alkaloide besitzen bis heute große Bedeutung in der kombinierten Chemotherapie myeloischer Tumore. Taxane haben in jüngster Zeit bedeutende Anwendungsgebiete erschlossen, die durch bisher verfügbare Zytostatika nicht zugänglich waren, z.B. Ovarialkarzinome, maligne Melanome. Die Nebenwirkungen der Taxane sind allerdings denen anderer Zytostatika vergleichbar (z.B. Haarausfall, sensorische Neuropathie). Multi-Drug-resistente Tumorzellen, die das P-Glycoprotein überexprimieren sind gegenüber Taxanen resistent. Die begrenzte Verfügbarkeit des Naturstoffs Taxol behindert zudem eine breitere klinische Testung.

Es wurden deshalb Naturstoffe und synthetische Pharmaka getestet, die ein von den bisherigen Mitosegiften abweichendes Wirkspektrum besitzen. Eine *in vitro* Versuchsanordnung ermöglicht die Suche nach Substanzen, die die GTP-abhängige Polymersiation von Tubulin nicht beeinflussen, die Depolymersiation der gebildeten Mikrotubuli jedoch verhindern. Substanzen mit einem solchen Wirkprofil sollten die vielseitigen Funktionen von Mikrotubuli in extranukleären Zellkompartimenten weniger stark beeinflussen, als die Dynamik des Spindelapparats während der Mitose (Meta-/Anaphase). Folgerichtig sollten solche Verbindungen geringere Nebenwirkungen *in vivo* zeigen als Taxane oder Vinka-Alkaloide.

Tubulin ist ein essentieller Bestandteil der mitotischen Spindel. Es dient unter anderem zur Aufrechterhaltung der Zellform, zum Transport von Organellen innerhalb der Zelle und zur Beeinflussung der Zellbeweglichkeit.

Taxane stellten bislang die einzige bekannte Strukturklasse dar, die in der Lage ist, die Polymerisation von Tubulin (überwiegend in der G2-Phase) zu beschleunigen sowie die gebildeten Mikrotubuli-Polymere zu stabilisieren. Dieser Mechanismus unterscheidet sich deutlich von denjenigen die andere, ebenfalls die phasenspezifische Zellteilung beeinflussende Strukturklassen aufweisen. So hemmen beispielsweise Substanzen aus der Gruppe der Vinka-Alkaloide (z.B. Vincristine und Vinblastine) aber auch Colchicin die Polymerisation der Tubulindimeren in der M-Phase.

Es wurde nun gefunden, daß vergleichsweise einfach herzustellende Verbindungen der Formel I in der Lage sind, die Depolymerisation von Mikrotubuli zu hemmen, ohne GTP-unabhängig die Bildung von Mikrotubuli zu steigern. Darüber hinaus wurden Verbindungen mit einem völlig neuen Wirkprofil identifiziert, die in der Lage sind, die Depolymerisation von Mikrotubuli zu beschleunigen. Auf Grund dieser Eigenschaften stellen die Verbindungen der Formel I wertvolle Pharmaka dar, die prinzipiell in der Lage sind, die schwer zu synthetisierenden und in ausreichenden Mengen noch nicht verfügbaren Taxane wie z.B. Taxol und Taxotere® in der Therapie maligner Tumore zu ergänzen bzw. zu ersetzen (EP-A 253739).

Die neuen Borneolderivate sind gekennzeichnet durch die allgemeine Formel I 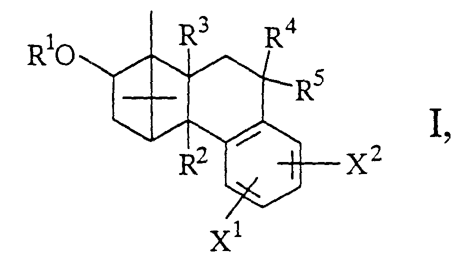 worin
R¹ C(O)-CH(OR⁶)-CH(NR^{7a}R^{7b})-R⁸, C(O)-CH(OR^{6a})-CH[NH(C(O)-CH(OR^{6b})-CH(NR^{7a}R^{7b})-R⁸)]-R⁸,
R² Wasserstoff, -OH, C₁-C₁₀-Alkyl, C₁-C₁₀-Alkoxy, -DC(O)R^{9a}, -OSO₂R^{9a}, -OP(O)(OH)₂, NHR^{9a}, NR^{9a}R^{9b},
R³ Wasserstoff, -OH, C₁-C₁₀-Alkoxy, -OC(O)R^{9b}, -OSO₂R^{9b}, -OP(O)(OH)₂, oder
R², R³ gemeinsam ein Sauerstoffatom,
R⁴ Wasserstoff, C₁-C₁₀-Alkyl, -(CH₂)ₙ-OR^{11a},
R⁵ Wasserstoff, C₁-C₁₀-Alkyl, -(CH₂)ₚ-OR^{11b}, oder
R⁴, R⁵ gemeinsam ein Sauerstoffatom, eine =CHR¹⁰-Gruppe,
R^{6a}, R^{6b} gleich oder verschieden sind und R⁶ bedeuten,
R^{7a}, R^{7b} gleich oder verschieden sind und R⁷ bedeuten,
n 0 bis 8
p 1 bis 8
R⁷ -C(O)R¹², -SO₂R¹², -C(O)OR¹², -C(O)SR¹², -C(O)NHR^{9d}, -C(O)NR^{9d}R^{9e}, C₁-C₁₀-Alkyl, 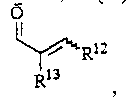
R⁸ Phenyl,
R^{9a-e}, R¹² gleich oder verschieden sind und C₁-C₁₀-Alkyl, C₄-C₈-Cycloalkyl, Aryl, C₇-C₁₆-Aralkyl bedeuten,
R¹⁰ Wasserstoff, C₁-C₁₀-Alkyl, -(CH₂)ₛ-OR¹⁴,
s 1 bis 8
R⁶, R^{11a,b}, R¹⁴ gleich oder verschieden sind und Wasserstoff, C₁-C₁₀-Alkyl, Aryl, C₁-C₁₀-Acyl, C₇-C₁₆-Aralkyl, -SO₂R^{9c}, -P(O)(OH)₂ bedeuten,
R¹³, R^{15a,b} gleich oder verschieden sind und Wasserstoff, C₁-C₁₀-Alkyl, Aryl, C₇-C₁₆-Aralkyl bedeuten,
X¹, X² gleich oder verschieden sind und X bedeuten,
X Wasserstoff, Halogen, -OH, -NO₂, -N₃, -CN, -NR^{15a}R^{15b}, -NHSO₂R^{15a}, -CO₂R¹⁵, C₁-C₁₀-Alkyl, C₁-C₁₀-Alkoxy, C₁-C₁₀-Acyloxy, C₁-C₁₀-Acyl sein kann
   und, falls R¹⁵ Wasserstoff bedeutet, deren Salze mit physiologisch verträglichen Basen, sowie deren α-, β- oder γ-Cyclodextrinclathrate, sowie die mit Liposomen verkapselten Verbindungen der allgemeinen Formel I bedeuten.

Die Erfindung betrifft die Diastereomeren und/oder Enantiomeren dieser Borneolderivate und auch deren Gemische.

Als Alkylgruppen R², R⁴, R⁵, R⁶, R^{9a-e}, R¹⁰, R^{11a,b}, R¹², R¹³, R¹⁴, R^{15a,b} und X sind gerad- oder verzweigtkettige Alkylgruppen mit 1-10 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.

Die Alkylgruppen R², R⁴, R⁵, R⁶, R^{9a-e}, R¹⁰, R^{11a,b}, R¹², R¹³, R¹⁴, R^{15a,b} und X können substituiert sein durch 1-3 Halogenatome, Hydroxygruppen, C₁-C₄-Alkoxygruppen, C₆-C₁₂-Arylgruppen, die durch 1-3 Halogenatome substituiert sein können, Di-(C₁-C₄)-Alkylamine und Tri-(C₁-C₄)-Alkylammonium.

Als Cycloalkylgruppen R^{9a-e}, R¹² kommen substituierte und unsubstituierte Reste mit 4 bis 8 Kohlenstoffatomen in Betracht.

Als Arylrest R⁶, R^{9a-e}, R¹⁰, R^{11a,b}, R¹², R¹³, R¹⁴, R^{15a,b} kommen substituierte und unsubstituierte carbocyclische oder heterocyclische Reste wie z.B. Phenyl, Naphtyl, Furyl, Thienyl, Pyridyl, Pyrazolyl, Pyrimidinyl, Oxazolyl, Pyridazinyl, Pyrazinyl, Chinolyl, die mehrfach substituiert sein können durch die in X definierten Gruppen, in Frage.

Die in R², R³ und X der allgemeinen Formel I enthaltenen Alkoxy-, Acyl- sowie Acyloxygruppen sollen jeweils 1 bis 10 Kohlenstoffatome enthalten, wobei Methoxy-, Ethoxy- Propoxy- Isopropoxy-, t-Butyloxy-, Formyl, Acetyl, Propionyl-, und Isopropionylgruppen bevorzugt sind.

Die C₇-C₁₆-Aralkylgruppen in R⁶, R^{9a-e}, R^{11a,b}, R¹², R¹³, R¹⁴, R^{15a,b} können im Ring bis 14 C-Atome enthalten, bevorzugt 6 bis 10 und in der Alkylkette 1 bis 4 , bevorzugt 1 bis 2 Atome. Bevorzugte Aralkylreste sind z.B. Benzyl, Phenylethyl, Naphtylmethyl bzw. Naphtylethyl. Die Ringe können mehrfach substituiert sein durch die in X definierten Gruppen.

Freie Hydroxygruppen in R¹, R², R³, R⁴, R⁵, R¹⁰ und X können funktionell abgewandelt sein, beispielsweise durch Veretherung oder Veresterung, wobei freie Hydroxygruppen bevorzugt sind.

Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, tert.-Butyldimethylsilyl-, tert.-Butyldiphenylsilyl-, Tribenzylsilylrest. Als Acylreste kommen z.B. Acetyl, Propionyl, Butyryl, Benzoyl in Frage.

Halogen in den Definitionen für X bedeutet Fluor, Chlor, Brom und Iod.

Zur Salzbildung mit den freien Säuren (R¹⁵ = H) sind anorganische und organische Basen geeignet, wie sie dem Fachmann zur Bildung physiologisch verträglicher Salze bekannt sind. Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w.

Die Erfindung betrifft ferner Verfahren zur Herstellung von Borneolderivaten der Formel I, welches dadurch gekennzeichnet, daß man ein Olefin der allgemeinen Formel II 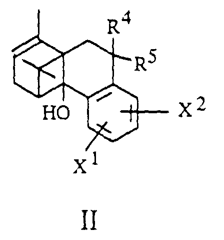 in dem R⁴, R⁵, X¹ und X² die oben genannten Bedeutungen haben und in X¹ oder X² enthaltene Hydroxylgruppen gegebenfalls geschützt sind, epoxidiert und das gebildete Epoxid ohne Isolation zu einem Alkohol der allgemeinen Formel III 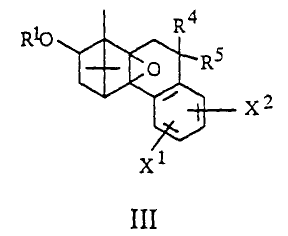 in dem R⁴, R⁵, X¹ und X² die oben genannten Bedeutungen haben und in R¹, X¹ oder X² enthaltene Hydroxylgruppen gegebenfalls geschützt sind, umlagert und dieses Umlagerungsprodukt in ein Derivat der allgemeinen Formel I überführt.

Die Reaktionsbedingungen der vorgenannten Verfahrensstufen sind:

### a) II → III

Die Epoxidierung der Doppelbindung erfolgt mit einer Peroxyverbindung wie z.B. meta-Chlorperbenzoesäure, Peroxotrifluoressigsäure, Wasserstoffperoxid, tert.-Butylhydro-peroxid gegebenfalls unter Zusatz einer Lewissäure wie z.B. Titantetraisopropoxid in einem inerten Solvens wie z.B. Dichlormethan, Toluol bei -40°C bis +40°C. Bevorzugt ist die Umsetzung mit tert.-Butylhydroperoxid und Titantetraisopropoxid in Toluol bei-10°C bis + 25°C.

Die Umlagerung des gebildeten Epoxides wird durch Säuren wie z.B. para-Toluolsulfonsäure, Kieselgel, saure Ionenaustauscherharze, Salzsäure katalysiert. Bevorzugt ist die Verwendung von Kieselgel.

### b) III → I

Die Überführung der Verbindungen der Formel III in eine Verbindung der Formel I kann in unterschiedlicher Reihenfolge vorgenommen werden:
1) Veresterung der Alkoholfunktion (R¹ = Wasserstoff) → Modifikation von R⁴ und/oder R⁵ → gegenenfalls Epoxidöffnung, falls R² und R³ gemeinsam ein Sauerstoffatom darstellen, gegebenfalls mit anschließender Modifikation von R² und R³.
2) Veresterung der Alkoholfunktion (R¹ = Wasserstoff) → gegenenfalls Epoxidöffnung, falls R² und R³ gemeinsam ein Sauerstoffatom darstellen, gegebenfalls mit anschließender Modifikation von R² und R³ → Modifikation von R⁴ und/oder R⁵.
3) Schutz der Alkoholfunktion (R¹ = Wasserstoff) → gegenenfalls Epoxidöffnung, falls R² und R³ gemeinsam ein Sauerstoffatom darstellen, gegebenfalls mit anschließender Modifikation von R² und R³ → Modifikation von R⁴ und/oder R⁵ → Freisetzung und anschließende Veresterung der Alkoholfunktion (R¹ = Wasserstoff).
4) Schutz der Alkoholfunktion (R¹ = Wasserstoff) → Modifikation von R⁴ und/oder R⁵ → Freisetzung und anschließende Veresterung der Alkoholfunktion (R¹ = Wasserstoff) → gegenenfalls Epoxidöffnung, falls R² und R³ gemeinsam ein Sauerstoffatom darstellen, gegebenfalls mit anschließender Modifikation von R² und R³.

Zur Veresterung der Alkoholfunktion (R¹ = Wasserstoff) wird mit einer Base wie z.B. Metallhydriden (z.B. Natriumhydrid), Alkalialkoholaten (z.B. Natriummethanolat, Kalium-tert.-butanolat), Alkalihexamethyldisilazan (z.B. Natriumhexamethyldisilazan), 1,5-Diazabicyclo[4.3.0]non-5-en (DBN), 1,8-Diazabicyclo[5.4.0]undec-7-en (DBU), Triethylamin, 4-(Dimethylamino)pyridin (DMAP), 1,4-Diazabicyclo[2.2.2]octan (DABCO) deprotoniert und mit geeigneten Carbonsäurederivaten wie z.B. Säureamiden, Säurehalogeniden, Säureanhydriden in einem inerten Solvens wie z.B. Dichlormethan, Diethylether, Tetrahydrofuran bei -70°C bis +50°C umgesetzt. Bevorzugt wird die Umsetzung mit Natriumhexamethyldisilazan als Base, einem cyclischen Säureamid als Carbonsäurederivat, Tetrahydrofuran als Solvens bei Temperaturen von -40°C bis +25°C.

Falls R⁴ und R⁵ gemeinsam eine =CHR¹⁰-Gruppe darstellen, kann die Funktionalisierung der olefinischen Doppelbindung nach den, dem Fachmann bekannten Methoden erfolgen. Beispielsweise kann Wasserstoff angelagert werden z.B. durch kat. Hydrierung, es können Hydroxylgruppen eingeführt werden durch Wasseranlagerung (Hydroborierung, Oxymercurierung) oder durch 1.2-Bishydroxylierung z.B. mit Osmiumtetroxid oder Kaliumpermanganat. Die Einführung einer Carbonylgruppe (R⁴, R⁵ stellen gemeinsam ein Sauerstoffatom dar) gelingt nach Spaltung der Doppelbindung z.B. durch Ozonolyse oder durch oxidative Spaltung eines 1.2-Diols. Eine derartig erzeugte Carbonylgruppe kann beispielsweise reduziert, alkyliert oder als Carbonylkomponente in einer Wittig-Reaktion zum Aufbau modifizierter =CHR¹⁰-Gruppen verwendet werden.

Falls R² und R³ gemeinsam ein Sauerstoffatom darstellen, kann das Epoxid durch Nucleophile wie beispielsweise Wasser, Carbonsäurederivate (Carbonsäuren, Carbonsäurehalogenide, Carbonsäureanhydride), Sulfonsäurederivate (Sulfonsäuren, Sulfonsäurehalogenide, Sulfonsäureanhydride), Amine, in Gegenwart von mineralischen oder organischen Säuren wie beispielsweise Chlorwasserstoff, para-Toluolsulfonsäure oder Lewissäuren wie beispielsweise Bortrifluoridetherat, Titantetraisopropoxid, Cerammoniumnitrat entweder in inerten oder als Nucleophil fungierenden Lösungsmitteln bei -70°C bis +50°C umgesetzt werden.

### Biologische Wirkungen und Anwendungsbereiche der neuen Bomeolderivate:

Die neuen Verbindungen der Formel I sind wertvolle Pharmaka. Sie interagieren mit Tubulin, indem sie gebildete Mikrotubuli stabilisieren und sind somit in der Lage, die Zellteilung phasenspezifisch zu beeinflussen. Dies betrifft vor allem schnell wachsende, neoplastische Zellen, deren Wachstum durch interzelluläre Regelmechnismen weitgehend unbeeinflußt ist. Wirkstoffe dieser Art sind prinzipiell geeignet zur Behandlung von Erkrankungen, bei denen die Beeinflussung der Zellteilung therapeutisch indiziert sein kann wie dies z.B. bei der Therapie des morbus Alzheimer, der Malaria, der Therapie von Erkrankungen, welche durch gram-negative Bakterien verursacht sind, sowie zur Behandlung maligner Tumoren der Fall ist. Als Anwendungsbereich für maligne Tumoren seien beispielweise genannt die Therapie von Ovarial-, Magen-, Colon-, Adeno-, Brust-, Lungen-, Kopf- und Nacken-Karzinomen, dem malignen Melanom, der akuten lymphozytären und myelocytären Leukämie. Die erfindungsgemäßen Verbindungen können alleine oder zur Erzielung additiver oder synergistischer Wirkungen in Kombination mit weiteren in der Tumortherapie anwendbaren Prinzipien und Substanzklassen verwendet werden.

Als Beispiele seien genannt die Kombination mit
O Platinkomplexen wie z.B. Cisplatin, Carboplatin,
O interkalierenden Substanzen z.B. aus der Klasse der Anthracycline wie z.B. Doxorubicin oder aus der Klasse der Antrapyrazole wie z.B. CI-941,
O mit Tubulin interagierenden Substanzen z.B. aus der Klasse der Vinka-Alkaloide wie z.B. Vincristin, Vinblastin oder aus der Klasse der Taxane wie z.B. Taxol, Taxotere oder aus der Klasse der Makrolide wie z.B. Rhizoxin oder andere Verbindungen wie z.B. Colchicin, Combretastatin A-4,
O DNA Topoisomeraseinhibitoren wie z.B. Camptothecin, Etoposid, Topotecan, Teniposid,
O Folat- oder Pyrimidin-Antimetaboliten wie z.B. Lometrexol, Gemcitubin,
O DNA alkylierenden Verbindungen wie z.B. Adozelesin, Dystamycin A,
O Inhibitoren von Wachstumsfaktoren (z.B. von PDGF, EGF, TGFβ, EGF) wie z.B. Somatostatin, Suramin, Bombesin-Antagonisten,
O Inhibitoren der Protein Tyrosin Kinase oder der Protein Kinasen A oder C wie z.B. Erbstatin, Genistein, Staurosporin, Ilmofosin, 8-Cl-cAMP,
O Antihormonen aus der Klasse der Antigestagene wie z.B. Mifepriston, Onapriston oder aus der Klasse der Antiöstrogene wie z.B. Tamoxifen oder aus der Klasse der Antiandrosene wie z.B. Cyproteronacetat,
O Metastasen inhibierenden Verbindungen z.B. aus der Klasse der Eicosanoide wie z.B. PGI₂. PGE₁, 6-Oxo-PGE₁ sowie deren stabiler Derivate (z.B. Iloprost, Cicaprost, Beraprost),
O Inhibitoren des transmembranären Ca²⁺-Influx wie z.B. Verapamil, Galopamil, Flunarizin, Diltiazem, Nifedipin, Nimodipin,
O Neuroleptika wie z.B. Chlorpromazin, Trifuoperazin, Thioridazin, Perphenazin,
O Lokalanästhetika wie z.B. Carbanilat-Ca7, Cinchocain, Carbanilat-Ca3, Articain, Carbanilat, Lidocain.
O die Angiogenese inhibierenden Substanzen wie z.B. anti-VEGF-Antikörpern, Endostatin B, Interferon α, AGM 1470,
O Inhibitoren der Zellproliferation bei Psoriasis, Kaposisarcom, Neuroblastom.

Die Erfindung betrifft auch Arzneimittel auf Basis der pharmazeutisch verträglichen, d.h. in den verwendeten Dosen nicht toxischen Verbindungen der allgemeinen Formel I, gegebenenfalls zusammen mit den üblichen Hilfs- und Trägerstoffen.

Die erfindungsgemäßen Verbindungen können nach an sich bekannten Methoden der Galenik zu pharmazeutischen Präparaten für die enterale, percutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens oder Myrj, Magnesiumstearat, wäßrigen oder nicht wäßrigen Trägern, Paraffinderivaten, Netz-, Dispergier-, Emulgier-, Konservierungsmitteln und Aromastoffen zur Geschmackskorrektur (z.B. etherischen Ölen) gemischt werden.

Die Erfindung betrifft somit auch pharmazeutische Zusammensetzungen, die als Wirkstoff zumindest eine erfindungsgemäße Verbindung enthalten. Eine Dosiseinheit enthält etwa 0,1-100 mg Wirkstoff(e). Die Dosierung der erfindungsgemäßen Verbindungen liegt beim Menschen bei etwa 0,1-1000 mg pro Tag.

Die nachfolgenden Ausführungsbeispiele dienen zur weiteren Erläuterung des erfindungsgemäßen Verfahrens.

### BEISPIEL 1

### [1R-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

150 mg (214 µmol) der nach Beispiel 1a dargestellten polaren Verbindung A löst man unter einer Atmosphäre aus trockenem Argon in 7 ml wasserfreiem Tetrahydrofuran, versetzt mit 0,29 ml einer 1,1 M Lösung von Tetrabutylammoniumfluorid in Tetrahydrofuran und rührt 1 Stunde bei 23°C. Man engt ein und reinigt den Rückstand durch Chromatographie an ca. 40 ml feinem Kieselgel mit einem Gradientensystem aus n-Hexan und Ethylacetat. Isoliert werden 113 mg (207 µmol, 97%) der Titelverbindung als farbloser Schaum.
¹H-NMR (DMSO-d6, 80°C): δ = 0,78 (3H), 0,91 (3H), 1,02 (3H), 1,32 (9H), 1,58 (1H), 2,17 (1H), 2,75 (2H), 3,08 (1H), 4,36 (1H), 4,93 (1H), 5,01 (1H), 5,07 (1H), 5,33 (1H), 5,49 (1H), 6,53 (1H), 7,20-7,38 (7H), 7,56 (2H) ppm.

### BEISPIEL 1a

### [1R-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,12,12-trimethyl-4a,10a-epoxy-1,4-methanophenanthren-2-ylester (B)

79 mg (280 µmol) des nach Beispiel 1b dargestellten Gemisches sowie 176 mg des nach Beispiel 1c dargestellten β-Lactams löst man unter einer Atmosphäre aus trockenem Argon in 30 ml wasserfreiem Tetrahydrofuran, versetzt bei -35°C mit 0,34 ml einer 1M Lösung von Natriumhexamethyldisilazan in Tetrahydrofuran und rührt 15 Minuten nach. Man gießt in gesättigte Ammoniumchloridlösung, extrahiert mehrfach mit Ethylacetat, wäscht die vereinigten organischen Extrakte mit Wasser und gesättigter Natriumchloridlösung und trocknet über Natriumsulfat. Den nach Filtration und Lösungsmittelabzug erhaltenen Rückstand trennt man durch Chromatographie an ca. 70 ml feinem Kieselgel mit einem Gradientensystem aus n-Hexan und Ethylacetat. Isoliert werden 26 mg (37 µmol, 13%) der Titelverbindung B als unpolare Komponente sowie 91 mg (130 *µ* mol, 46%) der Titelverbindung A als polare Komponente, jeweils als farbloser Schaum.
¹H-NMR (CDCl₃) von A: δ = 0,8-1,47 (39H), 1,71 (1H), 2,22 (1H), 2,64 (1H), 2,71 (1H), 3,03 (1H), 4,66 (1H), 4,96 (1H), 5,02 (1H), 5,16 (1H), 5,47 (1H), 5,92 (1H), 7,16-7,54 (9H) ppm.
¹H-NMR (CDCl₃) von B: δ = 0,8-1,47 (39H), 1,79 (1H), 2,38 (1H), 2,68 (1H), 2,70 (1H), 3,15 (1H), 4,69 (1H), 5,06 (1H), 5,08 (1H), 5,22 (1H), 5,50 (1H), 5,71 (1H), 7,18-7,56 (9H) ppm.

### BEISPIEL 1b

### [1R-(1α,2β,4α,4aβ,10aβ)]-9-Methylen-1,2,3,4,4a,9,10,10a-octahydro-1,12,12-trimethyl-4a,10a-epoxy-1,4-methanophenanthren-2-ol (A) und [1S-(1α,2β,4α,4aβ, 10aβ)]-9-Methylen-1,2,3,4,4a,9,10,10a-octahydro-1,12,12-trimethyl-4a,10a-epoxy-1,4-methanophenanthren-2-ol (B)

2,1g (7,88 mmol) eines ca. 7:3-Gemisches aus [4R-(4α,4aβ,10aα)]-9-Methylen-3,4,4a,9,10,10a-hexahydro-1,11,11-trimethyl-4,10a-methanophenanthren-4a-ol und [4S-(4α,4aβ,10aα)]-9-Methylen-3,4,4a,9,10,10a-hexahydro-1,11,11-trimethyl-4,10a-methanophenanthren-4a-ol, das man in Analogie zu dem im J. Am. Chem. Soc. 1992, auf Seite 5879 beschriebenen Verfahren hergestellt hat, löst man unter einer Atmosphäre aus trockenem Argon in 80 ml wasserfreiem Dichlormethan und kühlt auf 0°C. Man versetzt mit 2,4 ml Titan(IV)isopropylat, 1,5 ml einer 6,5 M wasserfreien Lösung von tert.-Butylhydroperoxid in Toluol und rührt 0,5 Stunden. Man gießt in Wasser, extrahiert mehrfach mit Diethylether, wäscht die vereinigten organischen Extrakte mit Wasser und gesättigter Natriumchloridlösung und trocknet über Natriumsulfat. Den nach Filtration und Lösungsmittelabzug erhaltenen Rückstand trennt man durch Chromatographie an ca. 200 ml feinem Kieselgel mit einem Gradientensystem aus n-Hexan und Ethylacetat. Isoliert werden 1,39 g (4,92 mmol, 62%) der Titelverbindungen A und B als kristalliner Feststoff. Durch Kristallisation aus Diisopropylether läßt sich das im Überschuß in dem Produktgemisch enthaltene Isomer A enantiomerenrein gewinnen.
¹H-NMR (CDCl₃): δ = 0,79 (3H), 0,88 (3H), 1,11 (3H), 1,58 (1H), 2,38 (1H), 2,68 (1H), 2,74 (1H), 3,08 (1H), 3,79 (1H), 3,97 (1H), 5,02 (1H), 5,48 (1H), 7,30 (2H), 7,51 (2H) ppm.

### BEISPIEL 1c

### (3R,4S)-1-[(1,1-dimethylethoxy)carbonyl]-3-triisopropylsilyloxy-4-phenyl-2-azetidinon

2,5 g (7,82 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, löst man unter einer Atmosphäre aus trockenem Argon in 50 ml wasserfreiem Dichlormethan, kühlt auf 0°C, versetzt mit 3,1 ml Triethylamin, 3,65 ml Pyrokohlensäure-di-tert.butylether sowie einer katalytischen Menge Dimethylaminopyridin. Man läßt auf 23°C erwärmen und 16 Stunden rühren. Man gießt in gesättigte Ammoniumchloridlösung, extrahiert mehrfach mit Diethylether, wäscht die vereinigten organischen Extrakte mit Wasser und gesättigter Natriumchloridlösung und trocknet über Natriumsulfat. Den nach Filtration und Lösungsmittelabzug erhaltenen Rückstand trennt man durch Chromatographie an ca. 300 ml feinem Kieselgel mit einem Gradientensystem aus n-Hexan und Ethylacetat. Isoliert werden 3,1 g (7,39 mmol, 94%) der Titelverbindung als kristalliner Feststoff.
¹H-NMR (CDCl₃): δ = 0,80-1,02 (21H), 1,40 (9H), 5,07 (1H), 5,17 (1H), 7,27-7,40 (5H) ppm.

### BEISPIEL 1d

### (3R,4S)-1-benzoyl-3-triisopropylsilyloxy-4-phenyl-2-azetidinon

5,0 g (15,6 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, löst man unter einer Atmosphäre aus trockenem Argon in 100 ml wasserfreiem Dichlormethan, kühlt auf 0°C, versetzt mit 6,2 ml Triethylamin, 3,85 ml Benzoylchlorid sowie einer katalytischen Menge Dimethylaminopyridin. Man läßt auf 23°C erwärmen und 16 Stunden rühren. Man gießt in gesättigte Ammoniumchloridlösung, extrahiert mehrfach mit Diethylether, wäscht die vereinigten organischen Extrakte mit Wasser und gesättigter Natriumchloridlösung und trocknet über Natriumsulfat. Den nach Filtration und Lösungsmittelabzug erhaltenen Rückstand trennt man durch Chromatographie an ca. 400 ml feinem Kieselgel mit einem Gradientensystem aus n-Hexan und Ethylacetat. Isoliert werden 6,43 g (15,2 mmol, 97%) der Titelverbindung als kristalliner Feststoff.
¹H-NMR (CDCl₃): δ = 0,80-1,07 (21H), 5,25 (1H), 5,43 (1H), 7,27-7,43 (5H), 7,48 (2H), 7,59 (1H), 8,03 (2H) ppm.

### BEISPIEL 1e

### (3R,4S)-1-Cyclohexylcarbonyl-3-triisopropylsilyloxy-4-phenyl-2-azetidinon

2,0 g (6,3 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Cyclohexancarbonsäurechlorid um und isoliert nach Aufarbeitung und Reinigung 2,6 g (6,1 mmol, 96%) der Titelverbindung.
¹H-NMR (CDCl₃): δ = 0,81-1,03 (21H), 1,12-2,04 (10H), 3,03 (1H), 5,14 (1H), 5,19 (1H), 7,18-7,37 (5H) ppm.

### BEISPIEL 1f

### (3R,4S)-1-Acetyl-3-triisopropylsilyloxy-4-phenyl-2-azetidinon

2,0 g (6,3 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Acetylchlorid um und isoliert nach Aufarbeitung und Reinigung 1,8 g (5,0 mmol, 79%) der Titelverbindung.
¹H-NMR (CDCl₃): δ = 0,77-1,04 (21H), 2,45 (3H), 5,17 (1H), 5,22 (1H), 7,20-7,39 (5H) ppm.

### BEISPIEL 1g

### (3R,4S)-1-(1-Oxopropyl)-3-triisopropylsilyloxy-4-phenyl-2-azetidinon

96 mg (300 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Propionsäureanhydrid um und isoliert nach Aufarbeitung und Reinigung 85 mg (226 µmol, 75%) der Titelverbindung.
¹H-NMR (CDCl₃): δ = 0,75-1,05 (21H), 1,18 (3H), 2,29 (2H), 5,14 (1H), 5,20 (1H), 7,20-7,39 (5H) ppm.

### BEISPIEL 1h

### (3R,4S)-1-(1-Oxobutyl)-3-triisopropylsilyloxy-4-phenyl-2-azetidinon

96 mg (300 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Buttersäurechlorid um und isoliert nach Aufarbeitung und Reinigung 103 mg (264 µmol, 88%) der Titelverbindung.
¹H-NMR (CDCl₃): δ = 0,75-1,07 (24H), 1,70 (2H), 2,76 (2H), 5,17 (1H), 5,20 (1H), 7,20-7,40 (5H) ppm.

### BEISPIEL 1i

### (3R,4S)-1-(1-Oxo-3-methylbutyl)-3-triisopropylsilyloxy-4-phenyl-2-azetidinon

96 mg (300 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von 3-Methylbuttersäurechlorid um und isoliert nach Aufarbeitung und Reinigung 106 mg (263 *µ*mol, 88%) der Titelverbindung.
¹H-NMR (CDCl₃): δ = 0,74-1,05 (27H), 2,20 (1H), 2,58 (1H), 2,75 (1H), 5,16 (1H), 5,20 (1H), 7,20-7,38 (5H) ppm.

### BEISPIEL 1k

### (3R,4S)-1-(1-Oxo-3,3-dimethylbutyl)-3-triisopropylsilyloxy-4-phenyl-2-azetidinon

96 mg (300 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von 3,3-Dimethylbuttersäurechlorid um und isoliert nach Aufarbeitung und Reinigung 105 mg (251 *µ*mol, 84%) der Titelverbindung.
¹H-NMR (CDCl₃): δ = 0,80-1,14 (30H), 2,62 (1H), 2,81 (1H), 5,15 (1H), 5,19 (1H), 7,22-7,38 (5H) ppm.

### BEISPIEL 1l

### (3R,4S)-1-(1-Oxo-3-phenylpropyl)-3-triisopropylsilyloxy-4-phenyl-2-azetidinon

96 mg (300 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von 3-Phenylpropionsäurechlorid um und isoliert nach Aufarbeitung und Reinigung 60 mg (133 *µ*mol, 44%) der Titelverbindung.
¹H-NMR (CDCl₃): δ = 0,78-1,05 (21H), 2,92-3,25 (4H), 5,12 (1H), 5,17 (1H), 7,14-7,38 (10H) ppm.

### BEISPIEL 1m

### (3R,4S)-1-(1,4-Dioxo-4-methoxybutyl)-3-triisopropylsilyloxy-4-phenyl-2-azetidinon

96 mg (300 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Bemsteinsäuremethylesterchlorid um und isoliert nach Aufarbeitung und Reinigung 24 mg (55 µmol, 18%) der Titelverbindung.
¹H-NMR (CDCl₃): δ = 0,75-1,06 (21H), 2,66 (2H), 3,09 (2H), 3,67 (3H), 5,18 (1H), 5,23 (1H), 7,20-7,38 (5H) ppm.

### BEISPIEL 1n

### (3R,4S)-1-(1,4-Dioxo-4-hydroxybutyl)-3-triisopropylsilyloxy-4-phenyl-2-azetidinon

96 mg (300 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Bernsteinsäureanhydrid um und isoliert nach Aufarbeitung 122 mg (300 µmol, 100%) der Titelverbindung, die man ohne Reinigung weiter umsetzt.
¹H-NMR (CDCl₃): δ = 0,75-1,08 (21H), 2,72 (2H), 3,08 (2H), 5,18 (1H), 5,23 (1H), 7,20-7,40 (5H) ppm.

### BEISPIEL 1o

### (3R,4S)-1-(1-Oxo-3-cyclopentylpropyl)-3-triisopropylsilyloxy-4-phenyl-2-azetidinon

200 mg (626 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von 3-Cyclopentyl-propionsäurechlorid um und isoliert nach Aufarbeitung und Reinigung 102 mg (230 µmol, 37%) der Titelverbindung.
¹H-NMR (CDCl₃): δ = 0,80-1,19 (23H), 1,42-1,86 (9H), 2,79 (2H), 5,15 (1H), 5,20 (1H), 7,20-7,38 (5H) ppm.

### BEISPIEL 1p

### (3R,4S)-1-(Methoxycarbonyl)-3-triisopropylsilyloxy-4-phenyl-2-azetidinon

200 mg (626 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Chlorameisensäuremethylester um und isoliert nach Aufarbeitung und Reinigung 153 mg (405 µmol, 65%) der Titelverbindung.
¹H-NMR (CDCl₃): δ = 0,78-1,06 (21H), 3,80 (3H), 5,12 (1H), 5,20 (1H), 7,22-7,40 (5H) ppm.

### BEISPIEL 1q

### (3R,4S)-1-(Thiomethylcarbonyl)-3-[3-oxa-pent-2(RS)-yloxy]-4-phenyl-2-azetidinon

200 mg (626 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Chlorthioameisensäure-S-methylester um und isoliert nach Aufarbeitung und Reinigung 224 mg (569 µmol, 91%) der Titelverbindung.
¹H-NMR (CDCl₃): δ = 0,82-1,05 (21H), 2,37 (3H), 5,23 (2H), 7,22-7,40 (5H) ppm.

### BEISPIEL 1r

### (3R,4S)-1-(Ethoxycarbonyl)-3-triisopropylsilyloxy-4-phenyl-2-azetidinon

319 mg (1 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Chlorameisensäureethylester um und isoliert nach Aufarbeitung und Reinigung 392 mg (1 mmol, 100%) der Titelverbindung.
¹H-NMR (CDCl₃): δ = 0,76-1,05 (21H), 1,27 (3H), 4,23 (2H), 5,11 (1H), 5,20 (1H), 7,22-7,40 (5H) ppm.

### BEISPIEL 1s

### (3R,4S)-1-(Thioethylcarbonyl)-3-[3-oxa-pent-2(RS)-yloxy]-4-phenyl-2-azetidinon

960 mg (3,0 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Chlorthioameisensäure-S-ethylester um und isoliert nach Aufarbeitung und Reinigung 1,11 g (2,72 mmol, 91%) der Titelverbindung.
¹H-NMR (CDCl₃): δ = 0,76-1,04 (21H), 1,28 (3H), 2,93 (2H), 5,21 (2H), 7,20-7,40 (5H) ppm.

### BEISPIEL 1t

### (3R,4S)-1-(Butoxycarbonyl)-3-triisopropylsilyloxy-4-phenyl-2-azetidinon

96 mg (300 µmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Chlorameisensäurebutylester um und isoliert nach Aufarbeitung und Reinigung 106 mg (252 µmol, 84%) der Titelverbindung.
¹H-NMR (CDCl₃): δ = 0,76-1,04 (26H), 1,27 (1H), 1,57 (1H), 3,92 (1H), 4,15 (1H), 5,10 (1H), 5,20 (1H), 7,22-7,40 (5H) ppm.

### BEISPIEL 2

### [1S-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 26 mg (37 µmol) der nach Beispiel 1a dargestellten unpolaren Verbindung B umgesetzt. Nach Aufarbeitung und Reinigung isoliert man 14 mg (25 µmol, 69%) der Titelverbindung als farbloses Öl.
¹H-NMR (CDCl₃): δ = 0,84 (3H), 0,96 (3H), 1,08 (3H), 1,42 (9H), 1,83 (1H), 2,40 (1H), 2,70 (1H), 2,72 (1H), 3,18 (1H), 3,21 (1H), 4,58 (1H), 5,02 (1H), 5,10 (1H), 5,28 (1H), 5,50 (1H), 5,55 (1H), 7,22-7,38 (9H) ppm.

### BEISPIEL 3

### [1R-[1α,2β(2R*,3S*),4α,4aβ,10aβ]]-3-[benzoylamino]-2-hydroxybenzenpropan-säure-9-methylen-1,2,3,4,4a,9,10,10a-octahydro-1,12,12-trimethyl-4a,10a-epoxy-1,4-methanophenanthren-2-ylester

100 mg (142 µmol) der nach Beispiel 3a dargestellten polaren Verbindung A setzt man in Analogie zu Beispiel 1 um und isoliert nach Aufarbeitung und Reinigung 66 mg (120 µmol, 85%) der Titelverbindung als farbloses Öl.
¹H-NMR (CDCl₃): δ = 0,82 (3H), 0,91 (3H), 0,98 (3H), 1,81 (1H), 2,29 (1H), 2,68 (1H), 2,71 (1H), 2,91 (1H), 3,59 (1H), 4,71 (1H), 4,88 (1H), 5,13 (1H), 5,40 (1H), 5,71 (1H), 7,18 (2H), 7,22-7,40 (6H), 7,40-7,59 (4H), 7,70 (1H), 7,87 (2H) ppm.

### BEISPIEL 3a

### [1R-[1α,2β(2R*,3S*),4α,4aβ,10aβ]]-3-[benzoylamino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*), 4α,4aβ,10aβ]]-3-[benzoylamino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester (B)

80 mg (283 µmol) des nach Beispiel 1b dargestellten Gemisches sowie 181 mg des nach Beispiel 1d dargestellten β-Lactams setzt man in Analogie zu Beispiel 1a um und isoliert nach Aufarbeitung und Reinigung 24 mg (34 µmol, 12%) der Titelverbindung B als unpolare Komponente sowie 100 mg (142 *µ*mol, 50%) der Titelverbindung A als polare Komponente, jeweils als farbloses Öl.
¹H-NMR (CDCl₃) von A: δ = 0,8-1,22 (30H), 1,72 (1H), 2,20 (1H), 2,64 (1H), 2,66 (1H), 2,95 (1H), 4,78 (1H), 4,92 (1H), 5,05 (1H), 5,42 (1H), 5,68 (1H), 7,17-7,58 (12H), 7,65 (1H), 7,92 (2H) ppm.
¹H-NMR (CDCl₃) von B: δ = 0,77 (3H), 0,80 (3H), 0,85-1,18 (24H), 1,65 (1H), 2,28 (1H), 2,53 (1H), 2,61 (1H), 2,79 (1H), 4,78 (1H), 5,02 (1H), 5,15 (1H), 5,46 (1H), 5,78 (1H), 7,11 (2H), 7,21-7,57 (10H), 7,74 (1H), 7,92 (2H) ppm.

### BEISPIEL 4

### [1S-[1α,2β(2R*,3S*),4α,4aβ,10aβ]]-3-[benzoylamino]-2-hydroxybenzenpropan-säure-9-methylen-1,2,3,4,4a,9,10,10a-octahydro-1,12,12-trimethyl-4a,10a-epoxy-1,4-methanophenanthren-2-ylester

24 mg (34 µmol) der nach Beispiel 3a dargestellten unpolaren Verbindung B setzt man in Analogie zu Beispiel 1 um und isoliert nach Aufarbeitung und Reinigung 10 mg (18 µmol, 54%) der Titelverbindung als farbloses Öl.
¹H-NMR (CDCl₃): δ = 0,81 (3H), 0,90 (6H), 1,76 (1H), 2,32 (1H), 2,63 (1H), 2,67 (1H), 3,00 (1H), 3,34 (1H), 4,69 (1H), 5,07 (1H), 5,12 (1H), 5,47 (1H), 5,90 (1H), 7,16-7,58 (13H), 7,87 (2H) ppm.

### BEISPIEL 5

### [1S-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a-methoxy-10a-hydroxy-1,4-methanophenanthren-2-ylester

22 mg (30 µmol) der nach Beispiel 5a dargestellten Verbindung setzt man in Analogie zu Beispiel 1 um und isoliert nach Aufarbeitung und Reinigung 9 mg (16 µmol, 52%) der Titelverbindung als farbloses Öl.
¹H-NMR (CDCl₃): δ = 0,65 (3H), 1,01 (6H), 1,43 (9H), 2,07 (1H), 2,41 (1H), 2,57 (1H), 2,73 (2H), 2,82 (3H), 3,27 (1H), 3,82 (1H), 4,51 (1H), 5,12 (1H), 5,21 (2H), 5,33 (1H), 6,23 (1H), 7,22-7,50 (9H) ppm.

### BEISPIEL 5a

### [1S-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a-methoxy-10a-hydroxy-1,4-methanophenanthren-2-ylester

20 mg (28 µmol) der nach Beispiel 1a dargestellten unpolaren Verbindung B löst man in einem Gemisch aus 0,2 ml Dichlormethan und 2 ml Methanol, versetzt mit 200 mg DOWEX 50 WX 4 und rührt unter einer Atmosphäre aus trockenem Argon 5 Stunden bei 23°C. Man filtriert, wäscht das Filtrat mit gesättigter Natriumhydrogencarbonatlösung, gesättigter Natriumchloridlösung und trocknet über Magnesiumsulfat. Nach Filtration und Lösungsmittelabzug isoliert man 12 mg (16 µmol, 57%) der Titelverbindung als farbloses Öl, den man ohne Reinigung weiter umsetzt.
¹H-NMR (CDCl₃): δ = 0,64 (3H), 0,80-1,09 (27H), 1,44 (9H), 2,09 (1H), 2,43 (1H), 2,57 (1H), 2,68 (1H), 2,75 (1H), 2,80 (3H), 3,70 (1H), 4,60 (1H), 5,10 (1H), 5,12 (1H), 5,20 (1H), 5,34 (1H), 6,08 (1H), 7,17-7,49 (9H) ppm.

### BEISPIEL 6

### [1R-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a-methoxy-10a-hydroxy-1,4-methanophenanthren-2-ylester

15,6 mg (21 µmol) der nach Beispiel 6a dargestellten Verbindung setzt man in Analogie zu Beispiel 1 um und isoliert nach Aufarbeitung und Reinigung 8,6 mg (15 µmol, 71%) der Titelverbindung als farbloses Öl.
¹H-NMR (CDCl₃): δ = 0,65 (3H), 1,01 (6H), 1,43 (9H), 2,17 (1H), 2,38 (1H), 2,58 (1H), 2,73 (2H), 2,86 (3H), 3,11 (1H), 3,92 (1H), 4,56 (1H), 5,11 (1H), 5,21 (2H), 5,35 (1H), 6,21 (1H), 7,20-7,50 (9H) ppm.

### BEISPIEL 6a

### [1R-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a-methoxy-10a-hydroxy-1,4-methanophenanthren-2-ylester

15 mg (21 µmol) der nach Beispiel 1a dargestellten polaren Verbindung A setzt man in Analogie zu Beispiel 5a um und isoliert nach Aufarbeitung 15,7 mg (21 µmol, 100%) der Titelverbindung als farbloses Öl, der ohne Reinigung weiter umgesetzt wird.
¹H-NMR (CDCl₃): δ = 0,63 (3H), 0,78-1,04 (27H), 1,43 (9H), 2,03 (1H), 2,30 (1H), 2,58 (1H), 2,68 (1H), 2,74 (1H), 2,80 (3H), 3,82 (1H), 4,69 (1H), 5,11 (1H), 5,14 (1H), 5,27 (1H), 5,34 (1H), 6,39 (1H), 7,16-7,48 (9H) ppm.

### BEISPIEL 7

### [1R-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a-ethoxy-10a-hydroxy-1,4-methanophenanthren-2-ylester

16 mg (21 µmol) der nach Beispiel 7a dargestellten Verbindung setzt man in Analogie zu Beispiel 1 um und isoliert nach Aufarbeitung und Reinigung 7,8 mg (13 µmol, 63%) der Titelverbindung als farbloses Öl.
¹H-NMR (CDCl₃): δ = 0,64 (3H), 1,01 (6H), 1,06 (3H), 1,41 (9H), 2,22-2,43 (2H), 2,58 (1H), 2,68-2,90 (3H), 2,93-3,13 (2H), 4,04 (1H), 4,56 (1H), 5,12 (1H), 5,18 (1H), 5,32 (2H), 6,36 (1H), 7,18-7,50 (9H) ppm.

### BEISPIEL 7a

### [1R-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a-ethoxy-10a-hydroxy-1,4-methanophenanthren-2-ylester

15 mg (21 µmol) der nach Beispiel 1a dargestellten polaren Verbindung A setzt man in Analogie zu Beispiel 5a unter Verwendung von Ethanol um und isoliert nach Aufarbeitung 16 mg (21 µmol, 100%) der Titelverbindung, die man ohne Reinigung weiter umsetzt, als farbloses Öl.
¹H-NMR (CDCl₃): δ = 0,63 (3H), 0,78-1,12 (30H), 1,43 (9H), 2,13-2,37 (2H), 2,57 (1H), 2,64-2,86 (3H), 3,08 (1H), 3,98 (1H), 4,69 (1H), 5,12 (2H), 5,33 (2H), 6,33 (1H), 7,15-7,48 (9H) ppm.

### BEISPIEL 8

### [1S-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a,10a-dihydroxy-1,4-methanophenanthren-2-ylester

6,5 mg (9,0 µmol) der nach Beispiel 8a dargestellten Verbindung setzt man in Analogie zu Beispiel 1 um und isoliert nach Aufarbeitung und Reinigung 3,5 mg (6,2 µmol, 69%) der Titelverbindung als farbloses Öl.
¹H-NMR (CDCl₃): δ = 0,69 (3H), 1,01 (3H), 1,07 (3H), 1,48 (9H), 2,26-2,49 (2H), 2,54 (1H), 2,76 (1H), 2,88 (1H), 3,25 (1H), 4,40 (1H), 4,54 (1H), 4,84 (1H), 5,08 (1H), 5,34 (1H), 5,37 (1H), 5,53 (1H), 5,65 (1H), 7,20-7,49 (9H) ppm.

### BEISPIEL 8a

### [1S-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a,10a-dihydroxy-1,4-methanophenanthren-2-ylester

14 mg (20 µmol) der nach Beispiel 1a dargestellten unpolaren Verbindung B löst man in 1,5 ml Tetrahydrofuran, versetzt mit 0,5 ml Wasser, 3 Tropfen einer 4N Salzsäure und rührt unter einer Atmosphäre aus Argon 16 Stunden bei 23°C. Man gießt in gesättigte Natriumhydrogencarbonatlösung, extrahiert mit Diethylether, wäscht mit gesättigter Natriumchloridlösung und trocknet über Magnesiumsulfat. Den nach Filtration und Lösungsmittelabzug erhaltenen Rücklstand reinigt man durch Chromatographie an einer analytischen Dünnschichtplatte. Als Laufmittel dient ein Gemisch aus n-Hexan und Ethylacetat, als Elutionsmittel Diethylether. Isoliert werden 4,2 mg (5,8 µmol, 29%) der Titelverbindung als farbloser Schaum sowie 8,6 mg Ausgangsmaterial.
¹H-NMR (CDCl₃): δ = 0,70 (3H), 0,80-1,08 (27H), 1,45 (9H), 2,31 (1H), 2,49 (1H), 2,54 (1H), 2,72 (1H), 2,86 (1H), 3,99 (1H), 4,54 (1H), 4,63 (1H), 5,08 (1H), 5,22-5,40 (3H), 5,75 (1H), 7,18-7,47 (9H) ppm.

### BEISPIEL 9

### [1R-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a,10a-dihydroxy-1,4-methanophenanthren-2-ylester

6,0 mg (8,3 µmol) der nach Beispiel 9a dargestellten Verbindung setzt man in Analogie zu Beispiel 1 um und isoliert nach Aufarbeitung und Reinigung 3,0 (5,3 µmol, 64%) der Titelverbindung als farbloses Öl.
¹H-NMR (CDCl₃): δ = 0,71 (3H), 1,04 (6H), 1,40 (9H), 2,22-2,48 (2H), 2,57 (1H), 2,73 (1H), 2,89 (1H), 3,01 (1H), 3,12 (1H), 3,95 (1H), 4,54 (1H), 5,12 (1H), 5,21 (2H), 5,37 (1H), 5,93 (1H), 7,22-7,50 (9H) ppm.

### BEISPIEL 9a

### [1R-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a,10a-dihydroxy-1,4-methanophenanthren-2-ylester

15 mg (21,4 µmol) der nach Beispiel 1a dargestellten polaren Verbindung A setzt man in Analogie zu Beispiel 8a um und isoliert nach Aufarbeitung und Reinigung 6 mg (8,3 µmol, 39%) der Titelverbindung als farbloses Öl.
¹H-NMR (CDCl₃): δ = 0,70 (3H), 0,78-1,50 (36H), 2,18-2,40 (2H), 2,56 (1H), 2,71 (1H), 2,86 (1H), 3,26 (1H), 4,25 (1H), 4,68 (1H), 5,12 (2H), 5,34 (2H), 5,99 (1H), 7,16-7,47 (9H) ppm.

### BEISPIEL 10

### [1R-[1α,2β(2R*,3S*),4α,4aβ,10aβ]]-3-[[Cyclohexylcarbonyl]amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

38 mg (53 µmol) der nach Beispiel 10a dargestellten Verbindung setzt man in Analogie zu Beispiel 1 um und isoliert nach Aufarbeitung und Reinigung 24 mg (43 µmol, 81%) der Titelverbindung als farbloses Öl.
¹H-NMR (CDCl₃): δ = 0,60 (1H), 0,87 (3H), 0,93 (3H), 0,97-1,85 (14H), 2,22 (1H), 2,35 (1H), 2,76 (1H), 2,82 (1H), 3,10 (1H), 4,53 (1H), 5,08 (1H), 5,10 (1H), 5,47 (1H), 5,54 (1H), 7,19-7,45 (8H), 7,52 (1H), 7,58 (1H) ppm.

### BEISPIEL 10a

### [1R-[1α,2β(2R*,3S*),4α,4aβ,10aβ]]-3-[[Cyclohexylcarbonyl]amino]-2-(triisopropylsilyloxy)benzenpropansäure-9-methylen-1,2,3,4,4a,9,10,10a-octahydro-1,12,12-trimethyl-4a,10a-epoxy-1,4-methanophenanthren-2-ylester

15 mg (53 µmol) der nach Beispiel 1b dargestellten und durch Kristallisation enantiomerenrein erhaltenen Verbindung A sowie 34 mg des nach Beispiel 1e dargestellten β-Lactams setzt man in Analogie zu Beispiel 1a um und isoliert nach Aufarbeitung 44 mg der Titelverbindung als Rohprodukt, das man ohne Reinigung weiter umsetzt.
¹H-NMR (CDCl₃): δ = 0,70-1,85 (40H), 2,22 (2H), 2,66 (1H), 2,78 (1H), 3,07 (1H), 4,64 (1H), 5,01 (1H), 5,09 (1H), 5,47 (1H), 5,53 (1H), 7,10-7,41 (9H), 7,49 (1H), 7,56 (1H) ppm.

### BEISPIEL 11

### [1R-[1α,2β(2R*,3S*),4α,4aβ,10aβ]]-3-[Acetylamino]-2-hydroxybenzenpropansäure-9-methylen-1,2,3,4,4a,9,10,10a-octahydro-1,12,12-trimethyl-4a,10a-epoxy-1,4-methanophenanthren-2-ylester

6,0 mg (9,3 µmol)
¹H-NMR (CDCl₃): δ = 0,86 (3H), 0,93 (3H), 1,08 (3H), 1,74 (1H), 1,97 (3H), 2,35 (1H), 2,77 (1H), 2,83 (1H), 2,97 (1H), 3,09 (1H), 4,48 (1H), 4,97 (1H), 4,99 (1H), 5,52 (1H), 5,56 (1H), 7,19-7,42 (7H), 7,56 (2H), 7,61 (1H) ppm.

### BEISPIEL 11a

### [1R-[1α,2β(2R*,3S*),4α,4aβ,10aβ]]-3-[Acetylamino]-2-(triisopropylsilyloxy)benzenpropansäure-9-methylen-1,2,3,4,4a,9,10,10a-octahydro-1,12,12-trimethyl-4a,10a-epoxy-1,4-methanophenanthren-2-ylester

5,6 mg (20 µmol) der nach Beispiel 1b dargestellten und durch Kristallisation enantiomerenrein erhaltenen Verbindung A sowie 22 mg des nach Beispiel 1f dargestellten β-Lactams setzt man in Analogie zu Beispiel 1a um und isoliert nach Aufarbeitung 28 mg der Titelverbindung als Rohprodukt, das man ohne Reinigung weiter umsetzt.
¹H-NMR (CDCl₃): δ = 0,81 (3H), 0,88 (3H), 0,95-1,30 (25H), 1,38 (1H), 1,98 (3H), 2,14 (1H), 2,65 (1H), 2,80 (1H), 3,08 (1H), 4,63 (1H), 4,93 (1H), 4,99 (1H), 5,50 (1H), 5,56 (1H), 7,15-7,38 (4H), 7,42-7,58 (4H), 7,67 (1H) ppm.

### BEISPIEL 12

### [1R-[1α,2β(2R*,3S*),4α,4aβ,10aβ]]-3-[(1-Oxopropyl)amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 11 mg (17 *µ* mol) der nach Beispiel 12a dargestellten Verbindung umgesetzt. Nach Aufarbeitung und Reinigung isoliert man 3,0 mg (6,0 *µ*mol, 35%) der Titelverbindung als farbloses Öl.
¹H-NMR (CDCl₃): δ = 0,85 (3H), 0,93 (3H), 1,04 (3H), 1,08 (3H), 1,73 (1H), 2,22 (2H), 2,34 (1H), 2,77 (1H), 2,85 (1H), 2,99 (1H), 3,09 (1H), 4,48 (1H), 4,94 (1H), 5,01 (1H), 5,54 (1H), 5,58 (1H), 7,18-7,62 (10H) ppm.

### BEISPIEL 12a

### [1R-[1α,2β(2R*,3S*),4α,4aβ,10aβ]]-3-[(1-Oxopropyl)amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

10 mg (35 µmol) der nach Beispiel 1b dargestellten Verbindung A sowie 20 mg des nach Beispiel 1g dargestellten β-Lactams setzt man in Analogie zu Beispiel 1a um und isoliert nach Aufarbeitung und Reinigung 11 mg (17 µmol, 48%) der Titelverbindung als farbloses Öl.
¹H-NMR (CDCl₃): δ = 0,81 (3H), 0,88 (3H), 0,95-1,28 (24H), 1,38 (1H), 2,15 (1H), 2,25 (2H), 2,64 (1H), 2,80 (1H), 3,07 (1H), 4,63 (1H), 4,93 (1H), 5,00 (1H), 5,52 (1H), 5,58 (1H), 7,15-7,60 (10H) ppm.

### BEISPIEL 13

### [1R-[1α,2β(2R*,3S*),4α,4aβ,10aβ]]-3-[(1-Oxobutyl)amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 7,0 mg (10 *µ* mol) der nach Beispiel 13a dargestellten Verbindung umgesetzt. Nach Aufarbeitung und Reinigung isoliert man 2,0 mg (3,9 *µ*mol, 39%) der Titelverbindung als farbloses Öl.
¹H-NMR (CDCl₃): δ = 0,73 (3H), 0,86 (3H), 0,93 (3H), 1,08 (3H), 1,54 (2H), 1,73 (1H), 2,18 (2H), 2,33 (1H), 2,75 (1H), 2,84 (1H), 3,03 (1H), 3,10 (1H), 4,49 (1H), 4,98 (1H), 5,03 (1H), 5,53 (1H), 5,57 (1H), 7,18-7,62 (10H) ppm.

### BEISPIEL 13a

### [1R-[1α,2β(2R*,3S*),4α,4aβ,10aβ]]-3-[(1-Oxobutyl)amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

10 mg (35 µmol) der nach Beispiel 1b dargestellten Verbindung A sowie 21 mg des nach Beispiel 1h dargestellten β-Lactams setzt man in Analogie zu Beispiel 1a um und isoliert nach Aufarbeitung und Reinigung 7 mg (10 µmol, 30%) der Titelverbindung als farbloses Öl.
¹H-NMR (CDCl₃): δ = 0,72-1,28 (33H), 1,49 (1H), 1,54 (2H), 2,13 (1H), 2,20 (2H), 2,64 (1H), 2,80 (1H), 3,07 (1H), 4,63 (1H), 4,93 (1H), 5,02 (1H), 5,52 (1H), 5,58 (1H), 7,13-7,60 (10H) ppm.

### BEISPIEL 14

### [1R-[1α,2β(2R*,3S*),4α,4aβ,10aβ]]-3-[(3-Methyl-1-oxobutyl)amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 12 mg (17 *µ* mol) der nach Beispiel 14a dargestellten Verbindung umgesetzt. Nach Aufarbeitung und Reinigung isoliert man 4,0 mg (7,6 *µ*mol, 44%) der Titelverbindung als farbloses Öl.
¹H-NMR (CDCl₃): δ = 0,63 (3H), 0,75 (3H), 0.86 (3H), 0,92 (3H), 1,08 (3H), 1,76 (1H), 1,92-2,18 (3H), 2,35 (1H), 2,76 (1H), 2,84 (1H), 2,98 (1H), 3,09 (1H), 4,52 (1H), 5,02 (1H), 5,07 (1H), 5,52 (1H), 5,55 (1H), 7,19-7,62 (10H) ppm.

### BEISPIEL 14a

### [1R-[1α,2β(2R*,3S*),4α,4aβ,10aβ]]-3-[(3-Methyl-1-oxobutyl)amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

10 mg (35 µmol) der nach Beispiel 1b dargestellten Verbindung A sowie 21 mg des nach Beispiel 1i dargestellten β-Lactams setzt man in Analogie zu Beispiel 1a um und isoliert nach Aufarbeitung und Reinigung 12 mg (18 µmol, 50%) der Titelverbindung als farbloses Öl.
¹H-NMR (CDCl₃): δ = 0,64-1,30 (36H), 1,43 (1H), 1,96-2,22 (4H), 2,65 (1H), 2,80 (1H), 3,07 (1H), 4,63 (1H), 4,97 (1H), 5,05 (1H), 5,51 (1H), 5,58 (1H), 7,14-7,64 (10H) ppm.

### BEISPIEL 15

### [1R-[1α,2β(2R*,3S*),4α,4aβ,10aβ]]-3-[(3,3-Dimethyl-1-oxobutyl)amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 12 mg (17 *µ* mol) der nach Beispiel 15a dargestellten Verbindung umgesetzt. Nach Aufarbeitung und Reinigung isoliert man 6,0 mg (11 *µ* mol, 67%) der Titelverbindung als farbloses Öl.
¹H-NMR (CDCl₃): δ = 0,73 (9H), 0,88 (3H), 0,94 (3H), 1,06 (3H), 1,81 (1H), 1,93 (1H), 2,21 (1H), 2,37 (1H), 2,76 (1H), 2,82 (1H), 2,93 (1H), 3,08 (1H), 4,52 (1H), 5,04 (1H), 5,08 (1H), 5,50 (1H), 5,53 (1H), 7,19-7,59 (10H) ppm.

### BEISPIEL 15a

### [1R-[1α,2β(2R*,3S*),4α,4aβ,10aβ]]-3-[(3,3-Dimethyl-1-oxobutyl)amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

10 mg (35 µmol) der nach Beispiel 1b dargestellten Verbindung A sowie 22 mg des nach Beispiel 1k dargestellten β-Lactams setzt man in Analogie zu Beispiel 1a um und isoliert nach Aufarbeitung und Reinigung 12 mg (17 µmol, 48%) der Titelverbindung als farbloses Öl.
¹H-NMR (CDCl₃): δ = 0,80 (9H), 0,85 (3H), 0,89 (3H), 1,01 (3H), 1,04-1,31 (21H), 1,58 (1H), 1,97 (1H), 2,20 (1H), 2,25 (1H), 2,68 (1H), 2,79 (1H), 3,08 (1H), 4,63 (1H), 5,02 (1H), 5,07 (1H), 5,49 (1H), 5,57 (1H), 7,13-7,61 (10H) ppm.

### BEISPIEL 16

### [1R-[1α,2β(2R*,3S*),4α,4aβ,10aβ]]-3-[(3-Phenyl-1-oxopropyl)amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 19 mg (26 *µ* mol) der nach Beispiel 16a dargestellten Verbindung umgesetzt. Nach Aufarbeitung und Reinigung isoliert man 9 mg (16 µmol, 60%) der Titelverbindung als farbloses Öl.
¹H-NMR (CDCl₃): δ = 0,83 (3H), 0,90 (3H), 1,04 (3H), 1,59 (1H), 2,25 (1H), 2,44 (1H), 2,59 (1H), 2,70-89 (4H), 2,98 (1H), 3,03 (1H), 4,44 (1H), 4,81 (1H), 4,96 (1H), 5,20 (1H), 5,53 (1H), 6,84 (2H), 7,00 (2H), 7,03-7,23 (6H), 7,38 (2H), 7,45-7,52 (3H) ppm.

### BEISPIEL 16a

### [1R-[1α,2β(2R*,3S*),4α,4aβ,10aβ]]-3-[(3-Phenyl-1-oxopropyl)amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

10 mg (35 µmol) der nach Beispiel 1b dargestellten Verbindung A sowie 60 mg des nach Beispiel 11 dargestellten β-Lactams setzt man in Analogie zu Beispiel 1a um und isoliert nach Aufarbeitung und Reinigung 19 mg (26 µmol, 73%) der Titelverbindung als farbloses Öl.
¹H-NMR (CDCl₃): δ = 0,75-1,32 (31H), 2,01 (1H), 2,48 (1H), 2,60 (1H), 2,64 (1H), 2,75 (1H), 2,84 (2H), 3,03 (1H), 4,56 (1H), 4,86 (1H), 4,93 (1H), 5,25 (1H), 5,54 (1H), 6,88 (2H), 7,00 (2H), 7,02 (2H), 7,05-7,30 (6H), 7,52 (2H), 7,64 (1H) ppm.

### BEISPIEL 17

### [1R-[1α,2β(2R*,3S*),4α,4aβ,10aβ]]-3-[(1,4-Dioxo-4-hydroxybutyl)amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 25 mg (35 *µ* mol) der nach Beispiel 17a dargestellten Verbindung umgesetzt. Nach Aufarbeitung und Reinigung isoliert man 3,0 mg (5,5 *µ*mol, 16%) der Titelverbindung als farbloses Öl.
¹H-NMR (CDCl₃): δ = 0,68 (3H), 1,01 (3H), 1,03 (3H), 2,15 (1H), 2,40 (1H), 2,53 (1H), 2,55-2,69 (4H), 2,72 (1H), 2,82 (1H), 4,53 (1H), 5,08 (1H), 5,09 (1H), 5,33 (1H), 5,54 (1H), 7,20-7,47 (9H), 7,60 (1H) ppm.

### BEISPIEL 17a

### [1R-[1α,2β(2R*,3S*),4α,4aβ,10aβ]]-3-[(1,4-Dioxo-4-hydroxybutyl)amino]-2-(triisopropylsilyloxy)benzenpropansäure-9-methylen-1,2,3,4,4a,9,10,10a-octahydro-1,12,12-trimethyl-4a,10a-epoxy-1,4-methanophenanthren-2-ylester

10 mg (35 µmol) der nach Beispiel 1b dargestellten Verbindung A sowie 36 mg des nach Beispiel In dargestellten β-Lactams setzt man in Analogie zu Beispiel 1a um und isoliert nach Aufarbeitung 49 mg der Titelverbindung als Rohprodukt, das man ohne Reinigung weiter umsetzt.

### BEISPIEL 18

### [1R-[1α,2β(2R*,3S*),4α,4aβ,9ξ,10aβ]]-3-[[(1,1-Dimethylethoxy)carbonyl]amino]-2-hydroxybenzenpropansäure-9-hydroxy-9-hydroxymethylen-1,2,3,4,4a,9,10,10a-octahydro-1,12,12-trimethyl-4a,10a-epoxy-1,4-methanophenanthren-2-ylester

In Analogie zu Beispiel 1a werden 5,5 mg (7,5 *µ* mol) der nach Beispiel 18a dargestellten Verbindung umgesetzt. Nach Aufarbeitung und Reinigung isoliert man 4,0 mg (6,9 *µ*mol, 92%) der Titelverbindung als farbloses Öl.
¹H-NMR (CDCl₃): δ = 0,83 (3H), 0,90 (3H), 1,11 (3H), 1,48 (9H), 1,65 (1H), 1,92 (1H), 2,32 (1H), 2,72 (1H), 3,02 (1H), 3,14 (1H), 3,19 (1H), 3,82 (1H), 3,95 (1H), 4,51 (1H), 4,73 (1H), 5,02 (1H), 5,47 (1H), 6,12 (1H), 7,20-7,58 (8H), 7,83 (1H) ppm.

### BEISPIEL 18a

### [1R-[1α,2β(2R*,3S*),4α,4aβ,9ξ,10aβ]]-3-[[(1,1-Dimethylethoxy)carbonyl]amino]-2-(triisopropylsilyloxy)benzenpropansäure-9-hydroxy-9-hydroxymethylen-1,2,3,4, 4a,9,10,10a-octahydro-1,12,12-trimethyl-4a,10a-epoxy-1,4-methanophenanthren-2-ylester

10 mg (14 µmol) der nach Beispiel 1a dargestellten Verbindung A löst man unter einer Atmosphäre aus trockenem Argon in 1,7 ml Aceton, versetzt mit 15 mg N-Methylmorpholino-N-oxid, 30 µl einer 2,5%igen Lösung von Osmiumtetroxid in t-Butanol, 3,3 ml Wasser und rührt bei 40°C zwei Tage. Man versetzt mit 170 mg Natriumthiosulfat, extrahiert mit Diethylether, wäscht die organische Phase mit Wasser und trocknet über Magnesiumsulfat. Den nach Filtration und Lösungsmittelabzug erhaltenen Rücklstand reinigt man durch Chromatographie an einer analytischen Dünnschichtplatte. Als Laufmittel dient ein Gemisch aus n-Hexan und Ethylacetat, als Elutionsmittel Diethylether. Isoliert werden 5,5 mg (7,5 µmol, 55%) der Titelverbindung als farbloser Schaum.
¹H-NMR (CDCl₃): δ = 0,77 (3H), 0,82-1,34 (27H), 1,42 (1H), 1,47 (9H), 1,98 (1H), 2,13 (1H), 2,60 (1H), 3,10 (1H), 3,20 (1H), 3,74 (1H), 3,98 (1H), 4,63 (1H), 4,78 (1H), 5,07 (1H), 5,40 (1H), 6,21 (1H), 7,17-7,35 (4H), 7,41 (1H), 7,48 (1H), 7,54 (2H), 7,80 (1H) ppm.

### BEISPIEL 19

### [1R-[1α,2β(2R*,3S*),4α,4aβ,9β,10aβ]]-3-[[(1,1-Dimethylethoxy)carbonyl]amino]-2-hydroxybenzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 11 mg (16 *µ* mol) der nach Beispiel 19a dargestellten Verbindung umgesetzt. Nach Aufarbeitung und Reinigung isoliert man 8,0 mg (14,6 µmol, 89%) der Titelverbindung als farbloses Öl.
¹H-NMR (CDCl₃): δ = 0,78 (3H), 0,90 (3H), 1,02 (3H), 1,13-1,48 (12H), 1,78 (1H), 2,03 (1H), 2,19 (1H), 2,30 (1H), 2,66 (1H), 3,04 (1H), 3,25 (1H), 4,54 (1H), 4,95 (1H), 5,20 (1H), 5,93 (1H), 7,10-7,35 (6H), 7,47 (3H) ppm.

### BEISPIEL 19a

### [1R-[1α,2β(2R*,3S*),4α,4aβ,9β,10aβ]]-3-[[(1,1-Dimethylethoxy)carbonyl]amino]-2-(triisopropylsilyloxy)benzenpropansäure-9-methyl-1,2,3,4,4a,9,10,10a-octahydro-1,12,12-trimethyl-4a,10a-epoxy-1,4-methanophenanthren-2-ylester

10 mg (14 µmol) der nach Beispiel 1a dargestellten Verbindung A löst man in 2 ml Ethanol, versetzt mit 2 mg Palladium auf Kohle (10%ig) und hydriert bei 1 at unter einer Atmosphäre aus Wasserstoff. Nach Aufnahme der theoretisch berechneten Menge wird vom Katalysator agfiltriert, das Filtrat eingeengt und der erhaltene Rücklstand durch Chromatographie an einer analytischen Dünnschichtplatte gereinigt. Als Laufmittel dient ein Gemisch aus n-Hexan und Ethylacetat, als Elutionsmittel Diethylether. Isoliert werden 2,0 mg (2,8 *µ*mol, 20%) der Titelverbindung als farbloser Schaum.
¹H-NMR (CDCl₃): δ = 0,75 (3H), 0,82-1,48 (39H), 1,68 (1H), 1,95-2,30 (3H), 2,60 (1H), 3,03 (1H), 4,63 (1H), 4,89 (1H), 5,21 (1H), 5,90 (1H), 7,08-7,50 (9H), ppm.

### BEISPIEL 20

### [1R-[1α,2β(2R*,3S*),4α,4aβ,10aβ]]-3-[3-Cyclopentyl-1-oxopropyl)amino]-2-hydroxybenzenpropansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-[3-Cyclopentyl-1-oxopropyl)amino]-2-hydroxybenzenpropansäure-9-methylen-1,2,3,4,4a,9,10,10a-octahydro-1,12,12-trimethyl-4a,10a-epoxy-1,4-methanophenanthren-2-ylester (B)

In Analogie zu Beispiel 1a werden 9,0 mg (12 *µ* mol) des nach Beispiel 20a dargestellten Diastereomerengemisches umgesetzt. Nach Aufarbeitung und Reinigung isoliert man 2,3 mg (4 µmol, 34%) der Titelverbindung A als polarere Komponente sowie 1,1 mg (2 µmol, 16%) der Titelverbindung B als unpolarere Komponente jeweils als farbloses Öl.
¹H-NMR (CDCl₃) von A: δ = 0,87 (3H), 0,75-1,00 (2H), 0,93 (3H), 1,08 (3H), 1,2-1,7 (9H), 1,77 (1H), 2,08-2,41 (3H), 2,76 (1H), 2,84 (1H), 3,00 (1H), 3,10 (1H), 4,51 (1H), 5,02 (1H), 5,03 (1H), 5,51 (1H), 5,55 (1H), 7,18-7,61 (10H), ppm.
¹H-NMR (CDCl₃) von B: δ = 0,73-1.02 (11H), 1,2-1,68 (9H), 1.80 (1H), 2,22 (2H), 2,37 (1H), 2,63 (1H), 2,70 (1H), 2,95 (1H), 3,18 (1H), 4,58 (1H), 5,03 (1H), 5,11 (1H), 5,47 (1H), 5,67 (1H), 6,95 (1H), 7,2-7,58 (9H), ppm.

### BEISPIEL 20a

### [1R-[1α,2β(2R*,3S*),4α,4aβ,10aβ]]-3-[3-Cyclopentyl-1-oxopropyl)amino]-2-(triisopropylsilyloxy) benzenpropansäure-9-methylen-1,2,3,4,4a,9,10,10a octahydro-1,12,12-trimethyl-4a,10a-epoxy-1,4-methanophenanthren-2-ylester (A) und [1S-[1α,2β(2R*,3S*),4α,4aβ,10aβ]]-3-[3-Cyclopentyl-1-oxopropyl)amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester (B)

20 mg (71 µmol) des nach Beispiel 1b dargestellten Diastereomerengemisches der Verbindungen A und B sowie 48 mg des nach Beispiel 1o dargestellten β-Lactams setzt man in Analogie zu Beispiel 1a um und isoliert nach Aufarbeitung und Reinigung 27 mg (37 µmol, 52%) eines Gemisches der beiden Titelverbindungen, das man ohne Trennung weiter umsetzt, als farbloses Öl.

### BEISPIEL 21

### [1R-[1α,2β[2R*,3S*(2R*,3S*)],4α,4aβ,10aβ]]-3-[[3-(1-Oxobutyl)amino-2-hydroxy-3-phenyl-1-oxopropyl]amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 9 mg (9,1 *µ* mol) der nach Beispiel 21a dargestellten Verbindung umgesetzt. Nach Aufarbeitung und Reinigung isoliert man 2 mg (2,9 *µ* mol, 32%) der Titelverbindung als farbloses Öl.
¹H-NMR (CDCl₃): δ = 0,82 (6H), 0,91 (3H), 1,01 (3H), 1,51 (2H), 1,76 (1H), 2,04 (2H), 2,28 (1H), 2,71-2,83 (2H), 3,03 (1H), 3,13 (1H), 3,53 (1H), 4,22 (1H), 4,42 (1H), 5,03 (1H), 5,10 (1H), 5,27 (1H), 5,42 (1H), 5,45 (1H), 6,62 (1H), 7,0-7,3 (10H), 7,38 (2H), 7,55 (2H), 7,93 (1H) ppm.

### BEISPIEL 21a

### [1R-[1α,2β[2R*,3S*(2R*,3S*)],4α,4aβ,10aβ]]-3-[[3-(1-Oxobutyl)amino-2-hydroxy-3-phenyl-1-oxopropyl]amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

28 mg (100 µmol) der nach Beispiel 1b dargestellten Verbindung A sowie 78 mg (2 Äquivalente) des nach Beispiel 1h dargestellten β-Lactams setzt man in Analogie zu Beispiel 1a um und isoliert nach Aufarbeitung und Reinigung 9,0 mg (9,1 µmol, 9%) der Titelverbindung als farbloses Öl sowie 53 mg der in Beispiel 11a beschriebenen Verbindung.
¹H-NMR (CDCl₃): δ = 0,73 (3H), 0,75 (3H), 0,85-1,35 (48H), 1,52-1,73 (3H), 2,14 (1H), 2,21 (2H), 2,61 (2H), 2,86 (1H), 4,16 (1H), 4,55 (1H), 4,72 (1H), 4,91 (1H), 5,22 (1H), 5,38 (1H), 5,40 (1H), 7,09-7,35 (13H), 7,48 (2H), 7,91 (1H) ppm.

### BEISPIEL 22

### [1R-[1α,2β[2R*,3S*(2R*,3S*)],4α,4aβ,10aβ]]-3-[[3-Acetylamino-2-hydroxy-3-phenyl-1-oxopropyl]amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 25 mg (26 µmol) der nach Beispiel 22a dargestellten Verbindung umgesetzt. Nach Aufarbeitung und Reinigung isoliert man 11 mg (17 µmol, 65%) der Titelverbindung als farbloses Öl.
¹H-NMR (CDCl₃): δ = 0,83 (3H), 0,91 (3H), 1,01 (3H), 1,81 (1H), 1,86 (3H), 2,28 (1H), 2,45 (1H), 2,75 (1H), 2,78 (1H), 3,12 (1H), 3,6 (1H), 4,27 (1H), 4,40 (1H), 5,05 (1H), 5,10 (1H), 5,30 (1H), 5,42 (1H), 5,46 (1H), 6,67 (1H), 6,98-7,41 (12H), 7,53 (2H), 7,93 (1H) ppm.

### BEISPIEL 22a

### [1R-[1α,2β[2R*,3S*(2R*,3S*)],4α,4aβ,10aβ]]-3-[[3-Acetylamino-2-hydroxy-3-phenyl-1-oxopropyl]amino]-2-(trüsopropylsilyloxy) benzenpropansäure-9-methylen-1,2,3,4,4a,9,10,10a-octahydro-1,12,12-trimethyl-4a,10a-epoxy-1,4-methanophenanthren-2-ylester

40 mg (142 µmol) der nach Beispiel 1b dargestellten Verbindung A sowie 152 mg (3 Äquivalente) des nach Beispiel 1f dargestellten β-Lactams setzt man in Analogie zu Beispiel 1a um und isoliert nach Aufarbeitung und Reinigung 25 mg (26 µmol, 18%) der Titelverbindung als farbloses Öl.
¹H-NMR (CDCl₃): δ = 0,75 (3H), 0,77 (3H), 0,89 (3H), 0,94-1,23 (42H), 1,61 (1H), 2,02 (3H), 2,15 (1H), 2,56-2,68 (2H), 2,86 (1H), 4,20 (1H), 4,57 (1H), 4,73 (1H), 4,91 (1H), 5,24 (1H), 5,37 (1H), 5,40 (1H), 7,10-7,35 (13H), 7,48 (2H), 7,89 (1H) ppm.

### BEISPIEL 23

### [1R-[1α,2β(2R*,3S*),4α,4aβ,10aβ]]-3-(Methoxycarbonyl)amino-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 2,8 mg (4,2 *µ* mol) der nach Beispiel 23a dargestellten Verbindung A umgesetzt. Nach Aufarbeitung und Reinigung isoliert man 2,0 mg (4,0 *µ*mol, 94%) der Titelverbindung als farbloses Öl.
¹H-NMR (CDCl₃): δ = 0,86 (3H), 0,92 (3H), 1.07 (3H), 1,75 (1H), 2,31 (1H), 2,73 (1H), 2,79 (1H), 3,06 (1H), 3,14 (1H), 3,65 (3H), 4,52 (1H), 4,89 (1H), 5,05 (1H), 5,2,6 (1H), 5,54 (1H), 6,62 (1H), 7,18-7,60 (9H) ppm.

### BEISPIEL 23a

### [1R-[1α,2β(2R*,3S*),4α,4aβ,10aβ]]-3-(Methoxycarbonyl)amino-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-(Methoxycarbonyl)amino-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester (B)

10 mg (35 µmol) eines Gemisches der nach Beispiel 1b dargestellten Verbindungen A und B sowie 20 mg des nach Beispiel 1p dargestellten β-Lactams setzt man in Analogie zu Beispiel 1a um und isoliert nach Aufarbeitung und Reinigung 2,8 mg (4,2 *µ*mol, 12%) der Titelverbindung A sowie 2,3 mg (3,5 µmol, 10%) der Titelverbindung B jeweils als farbloses Öl.
¹H-NMR (CDCl₃) von A: δ = 0,70-1,20 (30H), 1,50 (1H), 2,18 (1H), 2,63 (1H), 2,26 (1H), 3,11 (1H), 3,65 (3H), 4,66 (1H), 4,88 (1H), 5,03 (1H), 5,24 (1H), 5,52 (1H), 6,64 (1H), 7,14-7,37 (5H), 7,42-7,59 (4H) ppm.
¹H-NMR (CDCl₃) von B: δ = 0,72-1,10 (30H), 1,77 (1H), 2,37 (1H), 2,61-2,75 (2H), 3,05 (1H), 3,65 (3H), 4,71 (1H), 5,06 (1H), 5,08 (1H), 5,28 (1H), 5,52 (1H), 6,02 (1H), 7,20-7,59 (9H) ppm.

### BEISPIEL 24

### [1R-[1α,2β(2R*,3S*),4α,4aβ,10aβ]]-3-(Ethoxycarbonyl)amino-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 16 mg (23,7 *µ* mol) der nach Beispiel 24a dargestellten Verbindung umgesetzt. Nach Aufarbeitung und Reinigung isoliert man 7 mg (13,5 µmol, 57%) der Titelverbindung als farbloses Öl.
¹H-NMR (CDCl₃): δ = 0,85 (3H), 0,92 (3H), 1,07 (3H), 1,21 (3H), 1,77 (1H), 2,30 (1H), 2,71 (1H), 2,78 (1H), 3,11 (1H), 3,15 (1H), 4,11 (2H), 4,53 (1H), 4,93 (1H), 5,06 (1H), 5.27 (1H), 5,52 (1H), 6,38 (1H), 7,18-7,37 (5H), 7,42-7,60 (4H) ppm.

### BEISPIEL 24a

### [1R-[1α,2β(2R*,3S*),4α,4aβ,10aβ]]-3-(Ethoxycarbonyl)amino-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

50 mg (177 µmol) der nach Beispiel 1b dargestellten Verbindung A sowie 114 mg des nach Beispiel 1r dargestellten β-Lactams setzt man in Analogie zu Beispiel 1a um und isoliert nach Aufarbeitung und Reinigung 119 mg (176 µmol, 100%) der Titelverbindung als farbloses Öl.
¹H-NMR (CDCl₃): δ = 0,72-1,34 (33H), 1,53 (1H), 2,19 (1H), 2,63 (1H), 2,73 (1H), 3,10 (1H), 4,11 (2H), 4,67 (1H), 4,90 (1H), 5,03 (1H), 5,24 (1H), 5,52 (1H), 6,38 (1H), 7,12-7,35 (5H), 7,39-7,58 (4H) ppm.

### BEISPIEL 25

### [1R-[1α,2β(2R*,3S*),4α,4aβ,10aβ]]-3-(Butoxycarbonyl)amino-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 9 mg (13 µmol) der nach Beispiel 25a dargestellten Verbindung umgesetzt. Nach Aufarbeitung und Reinigung isoliert man 5 mg (9 µmol, 70%) der Titelverbindung als farbloses Öl.
¹H-NMR (CDCl₃): δ = 0,60-1,00 (9H), 1,07 (3H), 1,31 (2H), 1,58 (2H), 1,78 (1H), 2,30 (1H), 2,71 (1H), 2,77 (1H), 3,03-3,25 (2H), 3,81 (1H), 4,04 (1H), 4,54 (1H), 4,97 (1H), 5,06 (1H), 5,25 (1H), 5,51 (1H), 6,30 (1H), 7,16-7,60 (9H) ppm.

### BEISPIEL 25a

### [1R-[1α,2β(2R*,3S*),4α,4aβ,10aβ]]-3-(Butoxycarbonyl)amino-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

5 mg (18 µmol) der nach Beispiel 1b dargestellten Verbindung A sowie 12 mg des nach Beispiel 1t dargestellten β-Lactams setzt man in Analogie zu Beispiel 1a um und isoliert nach Aufarbeitung und Reinigung 9,5 mg (13,5 µmol, 75%) der Titelverbindung als farbloses Öl.
¹H-NMR (CDCl₃): δ = 0,75-1,18 (33H), 1,29 (2H), 1,48-1,66 (3H), 2,19 (1H), 2,63 (1H), 2,72 (1H), 3,08 (1H), 3,82 (1H), 4,04 (1H), 4,67 (1H), 4,91 (1H), 5,02 (1H), 5,22 (1H), 5,48 (1H), 6,31 (1H), 7,14-7,57 (9H) ppm.

### BEISPIEL 26

### [1R-[1α,2β(2R*,3S*),4α,4aβ,10aβ]]-3-(Methansulfonyl)amino-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 7 mg (10 µmol) der nach Beispiel 26a dargestellten Verbindung umgesetzt. Nach Aufarbeitung und Reinigung isoliert man 4,8 mg (9,2 *µ*mol, 90%) der Titelverbindung als farbloses Öl.
¹H-NMR (CDCl₃): δ = 0,86 (3H), 0,93 (3H), 1,13 (3H), 1,79 (1H), 2,39 (1H), 2,61 (3H), 2,73 (1H), 2,77 (1H), 2,95 (1H), 3,36 (1H), 4,65 (1H), 4,84 (1H), 4,99 (1H), 5,22 (1H), 5,52 (1H), 6,41 (1H), 7,20-7,40 (5H), 7,45-7,60 (4H) ppm.

### BEISPIEL 26a

### [1R-[1α,2β(2R*,3S*),4α,4aβ,10aβ]]-3-(Methansulfonyl)amino-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

20 mg (33 µmol) des nach Beispiel 26b dargestellten Amins löst man in 1 ml wasserfreiem Dichlormethan, versetzt bei 0°C unter einer Atmosphäre aus trockenem Argon mit 14 µl Triethylamin, 12 mg Dimethylaminopyridin, 7,7 µl Methansulfonsäurechlorid und rührt 1 Stunde. Man versetzt mit gesättigter Natriumhydrogencarbonatlösung, extrahiert mit Dichlormethan, wäscht die organische Phase mit gesättigter Natriumchloridlösung und trocknet über Magnesiumsulfat. Den nach Filtration und Lösungsmittelabzug erhaltenen Rückstand reinigt man durch Chromatographie an zwei analytischen Dünnschichtplatten. Als Laufmittel dient ein Gemisch aus n-Hexan und Ethylacetat, als Elutionsmittel ein Gemisch aus Diethylether und 2-Propanol. Isoliert werden 7 mg (10,3 µmol, 31%) der Titelverbindung als farbloses Öl.
¹H-NMR (CDCl₃): δ = 0,83 (3H), 0,91 (3H), 1,00-1,22 (24H), 1,61 (1H), 2,28 (1H), 2,68 (4H), 2,72 (1H), 3,31 (1H), 4,81 (1H), 4,83 (1H), 4,97 (1H), 5,18 (1H), 5,51 (1H), 6,51 (1H), 7,2-7,37 (5H), 7,46-7,59 (4H) ppm.

### BEISPIEL 26b

### [1R-[1α,2β(2R*,3S*),4α,4aβ,10aβ]]-3-Amino-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

Die Lösung von 60 mg (85 µmol) der nach Beispiel 1a dargestellten Verbindung in 0,6 ml Tetrahydrofuran und 0,6 ml Dioxan versetzt man bei 0°C mit 0,6 ml einer 4N HCl/Dioxan-Lösung. Man Läßt auf 23°C erwärmen und gibt nach 2 bzw. 3 Stunden jeweils 0,6 ml einer 4N HCl/Dioxan-Lösung nach. Nach einer weiteren Stunde gießt man in 5%ige Natriumhydroxidlösung, extrahiert mit Diethylether, wäscht die organische Phase mit Wasser und trocknet über Magnesiumsulfat. Den nach Filtration und Lösungsmittelabzug erhaltenen Rückstand reinigt man durch Chromatographie an 30 ml feinem Kieselgel (0,040-0.063 mesh). Als Laufmittel dient ein Gemisch aus n-Hexan und Ethylacetat, dem man 1% Triethylamin zusetzt. Isoliert werden 21 mg (35 µmol, 41%) der Titelverbindung als farbloses Öl.
¹H-NMR (CDCl₃): δ = 0.82 (3H), 0.85-1,13 (27H), 1,64 (1H), 1,85 (2H), 2,23 (1H), 2,64 (1H), 2,72 (1H), 3,06 (1H), 4,38 (1H), 4,61 (1H), 4,91 (1H), 5,03 (1H), 5,46 (1H), 7,12-7,55 (9H) ppm.

### BEISPIEL 27

### [1R-[1α,2β(2R*,3S*),4α,4aβ,10aβ]]-3-[[(4-Methylphenyl)sulfonyl]amino]-2-hydroxybenzenpropansäure-9-methylen-1,2,3,4,4a,9,10,10a-octahydro-1,12,12-trimethyl-4a,10a,epoxy-1,4-methanophenanthren-2-ylester

In Analogie zu Beispiel 1a werden 8 mg (10,6 *µ* mol) der nach Beispiel 27a dargestellten Verbindung umgesetzt. Nach Aufarbeitung und Reinigung isoliert man 4 mg (6,7 *µ* mol, 63%) der Titelverbindung als farbloses Öl.
¹H-NMR (CDCl₃): δ = 0,89 (3H), 0,92 (3H), 1,16 (3H), 1,69 (1H), 2,20 (3H), 2,32 (1H), 2,73 (1H), 2,81 (1H), 2,99 (1H), 3,41 (1H), 4,61 (1H), 4,82 (1H), 4,90 (1H), 5,29 (1H), 5,60 (1H), 6,53 (1H), 6,75 (2H), 6,97-7,11 (3H), 7,15 (2H), 7,30-7,43 (4H), 7,53 (1H), 7,62 (1H) ppm.

### BEISPIEL 27a

### [1R-[1α,2β(2R*,3S*),4α,4aβ,10aβ]]-3-[[(4-Methylphenyl)sulfonyl]amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 26a werden 15 mg (25 *µ* mol) der nach Beispiel 26b dargestellten Verbindung umgesetzt. Nach Aufarbeitung und Reinigung isoliert man 6 mg (7,9 *µ* mol, 32%) der Titelverbindung als farbloses Öl.
¹H-NMR (CDCl₃): δ = 0,83 (3H), 0,89 (3H), 0,96-1,17 (24H), 1,40 (1H), 2,16 (1H), 2,24 (3H), 2,63 (1H), 2,76 (1H), 3,35 (1H), 4,71-4,79 (2H), 4,85 (1H), 5,23 (1H), 5,58 (1H), 6,68 (1H), 6,88 (2H), 6,99-7,11 (3H), 7,20-7,40 (5H), 7,48 (3H) ppm.

### BEISPIEL 28

### [1R-[1α,2β(2R*,3S*),4α,4aβ,10aβ]]-3-[[(Ethylamino)carbonyl]amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 16 mg (24 *µ* mol) der nach Beispiel 28a dargestellten Verbindung umgesetzt. Nach Aufarbeitung und Reinigung isoliert man 7 mg (13,5 *µ* mol, 56%) der Titelverbindung als farbloses Öl.
¹H-NMR (CDCl₃): δ = 0,70 (3H), 1,03 (3H), 1,07 (3H), 1,12 (3H), 2,21 (1H), 2,42 (1H), 2,57 (1H), 2,66 (1H), 2,74 (1H), 2,86 (1H), 3,21 (2H), 4,58 (1H), 4,71 (1H), 5,08 (1H), 5,10 (1H), 5,34 (1H), 5,37 (1H), 6,20 (1H), 7,18-7,50 (9H) ppm.

### BEISPIEL 28a

### [1R-[1α,2β(2R*,3S*),4α,4aβ,10aβ]]-3-[[(Ethylamino)carbonyl]amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

Die Lösung von 45 mg (75 µmol) des nach Beispiel 26b dargestellten Amins in 2 ml wasserfreiem Dichlormethan versetzt man bei 0°C mit 42 µl Triethylamin, 36 mg Dimethylaminopyridin, 23 µl Ethylisocyanat und rührt 1 Stunde. Man reinigt direkt durch Chromatographie an zwei analytischen Dünnschichtplatten. Als Laufmittel dient ein Gemisch aus n-Hexan und Ethylacetat, als Elutionsmittel ein Gemisch aus Diethylether und 2-Propanol. Isoliert werden 20 mg (30 µmol, 40%) der Titelverbindung als farbloses Öl.

### BEISPIEL 29

### [1R-[1α,2β(2R*,3S*),4α,4aβ,10aβ]]-3-[[[(1-Methyl)ethylamino]carbonyl]amino)-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 10 mg (15 *µ* mol) der nach Beispiel 29a dargestellten Verbindung umgesetzt. Nach Aufarbeitung und Reinigung isoliert man 4 mg (7,5 *µ* mol, 50%) der Titelverbindung als farbloses Öl.
¹H-NMR (CDCl₃): δ = 0,53 (3H), 0,88 (6H), 0,95 (3H), 1,11 (3H), 1,85 (1H), 2,47 (1H), 2,71 (1H), 2,82 (1H), 2,87 (1H), 3,20 (1H), 3,67 (1H), 4,58 (1H), 4,78 (1H), 5,04 (1H), 5,17 (1H), 5,21 (1H), 5,57 (1H), 6,66 (1H), 7,19-7,42 (7H), 7,58 (2H) ppm.

### BEISPIEL 29a

### [1R-[1α,2β(2R*,3S*),4α,4aβ,10aβ]]-3-[[[(1-Methyl)ethylamino]carbonyl]amino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 28a werden 20 mg (33 *µ* mol) des nach Beispiel 26b dargestellten Amins umgesetzt. Nach Aufarbeitung und Reinigung isoliert man 10 mg (14,5 *µ* mol, 44%) der Titelverbindung als farbloses Öl.
¹H-NMR (CDCl₃): δ = 0,57 (3H), 0,76-1,22 (33H), 1,70 (1H), 2,32 (1H), 2,76 (1H), 2,84 (1H), 3,20 (1H), 3,70 (1H), 4,72 (1H), 4,77 (1H), 4,98 (1H), 5,17 (1H), 5,20 (1H), 5,56 (1H), 6,56(1H), 7,12-7,42 (7H), 7,56 (2H) ppm.

### BEISPIEL 30

### [1S-[1α,2β(2R*,3S*),4α,4aβ,10aβ]]-3-(Methoxycarbonyl)amino-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 2,3 mg (3,5 *µ* mol) der nach Beispiel 23a dargestellten Verbindung B umgesetzt. Nach Aufarbeitung und Reinigung isoliert man 1,6 mg (3,2 µmol, 91%) der Titelverbindung als farbloses Öl.
¹H-NMR (CDCl₃): δ = 0,84 (3H), 0,95 (3H), 1,05 (3H), 1,81 (1H), 2,38 (1H), 2,70 (1H), 2,71 (1H), 3,15 (1H), 3,21 (1H), 3,66 (3H), 4,58 (1H), 5,03 (1H), 5,09 (1H), 5,32 (1H), 5,50 (1H), 5,79 (1H), 7,20-7,56 (9H) ppm.

### BEISPIEL 31

### [1R-[1α,2β(2R*,3S*),4α,4aβ,10aβ]]-3-(Methylthiocarbonyl)amino-2-hydroxybenzenpropansäure-9-methylen-1,2,3,4,4a,9,10,10a-octahydro-1,12,12-trimethyl-4a-ethoxy-10a-hydroxy-1,4-methanophenanthren-2-ylester (A) und [1S-[1α,2β(2R*,3S*),4α,4aβ,10aβ]]-3-(Methylthiocarbonyl)amino-2-hydroxybenzenpropansäure-9-methylen-1,2,3,4,4a,9,10,10a-octahydro-1,12,12-trimethyl-4a-ethoxy-10a-hydroxy-1,4-methanophenanthren-2-ylester (B)

Die Lösung von 32 mg (54 µmol) des nach Beispiel 31a dargestellten Gemisches in 1 ml Ethanol versetzt man mit 40 µl einer 1N wässrigen HCl-Lösungund rührt bei 23°C 1 Stunde. Man versetzt mit gesättigter Natriumhydrogencarbonatlösung, extrahiert mit Dichlormethan, wäscht die organische Phase mit gesättigter Natriumchloridlösung und trocknet über Magnesiumsulfat. Den nach Filtration und Lösungsmittelabzug erhaltenen Rückstand reinigt man durch Chromatographie an zwei analytischen Dünnschichtplatten. Als Laufmittel dient ein Gemisch aus n-Hexan und Ethylacetat, als Elutionsmittel ein Gemisch aus Diethylether und 2-Propanol. Nach Aufarbeitung und Reinigung isoliert man 3,2 mg (5,7 µmol, 10%) der Titelverbindung A sowie *2,5* mg (4,4 µmol, 8%) der Titelverbindung B jeweils als farbloses Öl.
¹H-NMR (CDCl₃) von A: δ = 0,64 (3H), 0,97 (3H), 1,02 (3H), 1,03 (3H), 2,03 (1H), 2,33 (3H), 2,40 (1H), 2,67 (1H), 2,71 (1H), 2,77 (1H), 2,83 (1H), 3,00 (1H), 3,19 (1H), 4,24 (1H), 4,50 (1H), 5,14 (1H), 5,26 (1H), 5,37 (1H), 5,50 (1H), 7,20-7,50 (9H), 7,72 (1H) ppm.
¹H-NMR (CDCl₃) von B: δ = 0,63 (3H), 0,93-1,12 (9H), 2,14 (1H), 2,35 (3H), 2,39 (1H), 2,70 (2H), 2,77 (1H), 2,86 (1H), 3,01 (1H), 3,08 (1H), 4,18 (1H), 4,53 (1H), 5,13 (1H), 5,21 (1H), 5,36 (1H), *5,55* (1H), 7,20-7,48 (9H), 7,58 (1H) ppm.

### BEISPIEL 31a

### [1R-[1α,2β(2R*,3S*),4α,4aβ,10aβ]]-3-(Methylthiocarbonyl)amino-2-(3-oxa-pent-2-yloxy)benzenpropansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-(Methylthiocarbonyl)amino-2-(3-oxa-pent-2-yloxy)benzenpropansäure-9-methylen-1,2,3,4,4a,9,10,10a-octahydro-1,12,12-trimethyl-4a,10a-epoxy-1,4-methanophenanthren-2-ylester (B)

30 mg (106 µmol) der nach Beispiel 1b dargestellten Verbindung sowie 49 mg des nach Beispiel 1q dargestellten β-Lactams setzt man in Analogie zu Beispiel 1a um und isoliert nach Aufarbeitung und Reinigung 24 mg (41 µmol, 38%) der Titelverbindungen als farbloses Öl.

### BEISPIEL 32

### [1R-[1α,2β(2R*,3S*),4α,4aβ,10aβ]]-3-(Ethylthiocarbonyl)amino-2-hydroxybenzenpropansäure-9-methylen-1,2,3,4,4a,9,10,10a-octahydro-1,12,12-trimethyl-4a-ethoxy-10a-hydroxy-1,4-methanophenanthren-2-ylester (A) und [1S-[1α,2β(2R*,3S*),4α,4aβ,10aβ]]-3-(Ethylthiocarbonyl)amino-2-hydroxybenzenpropansäure-9-methylen-1,2,3,4,4a,9,10,10a-octahydro-1,12,12-trimethyl-4a-ethoxy-10a-hydroxy-1,4-methanophenanthren-2-ylester (B)

In Analogie zu Beispiel 1a werden 22 mg (36 *µ* mol) des nach Beispiel 32a dargestellten Gemisches umgesetzt. Nach Aufarbeitung und Reinigung isoliert man 2,5 mg (4,3 *µ* mol, 12%) der Titelverbindung A sowie 3 mg (5,2 *µ*mol, 14%) der Titelverbindung B jeweils als farbloses Öl.
¹H-NMR (CDCl₃) von A: δ = 0,63 (3H), 0,95 (3H), 1,00 (3H), 1,02 (3H), 1,27 (3H), 2,03 (1H), 2,39 (1H), 2,66 (1H), 2,70 (1H), 2,76 (1H), 2,78-3,07 (4H), 3,21 (1H), 4,21 (1H), 4,49 (1H), 5,14 (1H), 5,27 (1H), 5,36 (1H), 5,50(1H), 7,21-7,48 (9H), 7,61 (1H) ppm.
¹H-NMR (CDCl₃) von B: δ = 0,64 (3H), 0,96-1,10 (9H), 1,29 (3H), 2,16 (1H), 2,38 (1H), 2,70 (1H), 2,77 (1H), 2,80-3,03 (5H), 3,07 (1H), 4,17 (1H), 4,54 (1H), 5,12 (1H), 5,21 (1H), 5,34 (1H), 5,54 (1H), 7,21-7,55 (10H) ppm.

### BEISPIEL 32a

### [1R-[1α,2β(2R*,3S*),4α,4aβ,10aβ]]-3-(Ethylthiocarbonyl)amino-2-(3-oxa-pent-2-yloxy)benzenpropansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-(Ethylthiocarbonyl)amino-2-(3-oxa-pent-2-yloxy)benzenpropansäure-9-methylen-1,2,3,4,4a,9,10,10a-octahydro-1,12,12-trimethyl-4a,10a-epoxy-1,4-methanophenanthren-2-ylester (B)

20 mg (71 µmol) der nach Beispiel 1b dargestellten Verbindung sowie 34 mg des nach Beispiel 1s dargestellten β-Lactams setzt man in Analogie zu Beispiel 1a um und isoliert nach Aufarbeitung und Reinigung 22 mg (36 µmol, 51%) der Titelverbindungen als farbloses Öl.

### BEISPIEL 33

### [1R-[1α,2β(2R*,3S*),4α,4aβ,9β,10aβ]]-3-(Methoxycarbonyl)amino-2-hydroxybenzenpropansäure-9-methyl-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,9β,10aβ]]-3-(Methoxycarbonyl)amino-2 hydroxybenzenpropansäure-9-methyl-1,2,3,4,4a,9,10,10a-octahydro-1,12,12-trimethyl-4a,10a-epoxy-1,4-methanophenanthren-2-ylester (B)

In Analogie zu Beispiel 1a werden 16 mg (24 µmol) des nach Beispiel 33a dargestellten Gemisches umgesetzt. Nach Aufarbeitung und Reinigung isoliert man 3 mg (6 µmol, 25%) der Titelverbindung A sowie 3 mg (6 µmol, 25%) der Titelverbindung B jeweils als farbloses Öl.
¹H-NMR (CDCl₃) von A: δ = 0,79 (3H), 0,89 (3H), 1,02 (3H), 1,42 (3H), 1,73 (1H), 2,09 (1H), 2,17 (1H), 2,28 (1H), 2,69 (1H), 3,11 (2H), 3,62 (3H), 4,54 (1H), 4,92 (1H), 5,28 (1H), 6,46 (1H), 7,10-7,59 (9H) ppm.
¹H-NMR (CDCl₃) von B: δ = 0,78 (3H), 0,92 (6H), 1,38 (3H), 1,83 (1H), 2,01 (1H), 2,21 (1H), 2,33 (1H), 2,68 (111), 3,04 (1H), 3,20 (1H), 3,63 (3H), 4,55 (1H), 5,13 (1H), 5,33 (1H), 6,01 (1H), 7,11-7,51 (9H) ppm.

### BEISPIEL 33a

### [1R-[1α,2β(2R*,3S*),4α,4aβ,9β,10aβ]]-3-(Methoxycarbonyl)amino-2-(triisopropylsilyloxy) benzenpropansäure-9-methyl-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,9β,10aβ]]-3-(Methoxycarbonyl)amino-2-(triisopropylsilyloxy) benzenpropansäure-9-methyl-1,2,3,4,4a,9,10,10a-octahydro-1,12,12-trimethyl-4a,10a-epoxy-1,4-methanophenanthren-2-ylester (B)

In Analogie zu Beispiel 19a werden 26 mg (39 *µ* mol) des nach Beispiel 23a dargestellten Gemisches umgesetzt. Nach Aufarbeitung und Reinigung isoliert man 16 mg (24 *µ* mol, 62%) der Titelverbindungen als farbloses Öl.

### BEISPIEL 34

### [1R-[1α,2β(2R*,3S*),4α,4aβ,9α,10aβ]]-3-(Ethoxycarbonyl)amino-2-hydroxybenzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 2 mg (3 µmol) der nach Beispiel 34a dargestellten Verbindung A umgesetzt. Nach Aufarbeitung und Reinigung isoliert man 1,3 mg (2,5 µmol, 85%) der Titelverbindung als farbloses Öl.
¹H-NMR (CDCl₃): δ = 0,78 (3H), 0,90 (3H), 1,07 (3H), 1,21 (3H), 1,43 (3H), 1,60 (1H), 1,75 (1H), 2,30 (1H), 2,47 (1H), 2,74 (1H), 3,00 (1H), 3,15 (1H), 4,10 (2H), 4,52 (1H), 4,83 (1H), 5,28 (1H), 6,89 (1H), 7,13-7,58 (9H) ppm.

### BEISPIEL 34a

### [1R-(1α,2β(2R*,3S*),4α,4aβ,9α.10aβ]]-3-(Ethoxycarbonyl)amino-2-(triisopropylsilyloxy) benzenpropansäure-9-methyl-1,2,3,4,4a,9,10,10a-octahydro 1,12,12-trimethyl-4a,10a-epoxy-1,4-methanophenanthren-2-ylester (A) und [1R-[1α,2β(2R*,3S*),4α,4aβ,9β,10aβ]]-3-(Ethoxycarbonyl)amino-2-(triisopropylsilyloxy) benzenpropansäure-9-methyl-1,2,3,4,4a,9,10,10a-octahydro-1,12,12-trimethyl-4a,10a-epoxy-1,4-methanophenanthren-2-ylester (B)

In Analogie zu Beispiel 19a werden 102 mg (151 *µ* mol) der nach Beispiel 24a dargestellten Verbindung umgesetzt. Nach Aufarbeitung und Reinigung isoliert man 2 mg (3 µmol, 2%) der Titelverbindung A sowie 38 mg (56 µmol, 37%) der Titelverbindung B jeweils als farbloses Öl.
¹H-NMR (CDCl₃) von A: δ = 0,72 (3H), 0,75-1,60 (35H), 2,15 (1H), 2,42 (1H), 2,63 (1H), 3,13 (1H), 4,12 (2H), 4,68 (1H), 4,82 (1H), 5,27 (1H), 6,90 (1H), 7,16-7,58 (9H) ppm.
¹H-NMR (CDCl₃) von B: δ = 0,75 (3H), 0,80-1,30 (31H), 1,42 (3H), 1,54 (1H), 2,05 (1H), 2,18 (1H), 2,59 (1H), 3,04 (1H), 4,11 (2H), 4,65 (1H), 4,89 (1H), 5,29 (1H), 6,27 (1H), 7,10-7,56 (9H) ppm.

### BEISPIEL 35

### [1R-[1α,2β(2R*,3S*),4α,4aβ,9β,10aβ]]-3-(Ethoxycarbonyl)amino-2-hydroxybenzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 38 mg (56 *µ* mol) der nach Beispiel 35a dargestellten Verbindung B umgesetzt. Nach Aufarbeitung und Reinigung isoliert man 26 mg (50 *µ*mol, 89%) der Titelverbindung als farbloses Öl.
¹H-NMR (CDCl₃): δ = 0,79 (3H), 0,90 (3H), 1,03 (3H), 1,20 (3H), 1,41 (3H), 1,73 (1H), 2,08 (1H), 2,20 (1H), 2,28 (1H), 2,68 (1H), 3,07 (1H), 3,20 (1H), 4,10 (2H), 4,52 (1H), 4,92 (1H), 5,28 (1H), 6,30 (1H), 7,10-7,60 (9H) ppm.

### BEISPIEL 36

### [1R-[1α,2β(2R*,3S*),4α,4aβ,9β,10aβ]]-3-(Butoxycarbonyl)amino-2-hydroxybenzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 7 mg (10 µmol) der nach Beispiel 36a dargestellten Verbindung umgesetzt. Nach Aufarbeitung und Reinigung isoliert man 5 mg (9 µmol, 90%) der Titelverbindung als farbloses Öl.
¹H-NMR (CDCl₃): δ = 0,60-1,62 (19H), 1,74 (1H), 2,07 (1H), 2,19 (1H), 2,30 (1H), 2,68 (1H), 3,07 (1H), 3,19 (1H), 3,80 (1H), 4,03 (1H), 4,53 (1H), 4,93 (1H), 5,27 (1H), 6,25 (1H), 7,10-7,58 (9H) ppm.

### BEISPIEL 36a

### [1R-[1α,2β(2R*,3S*),4α,4aβ,9β,10aβ]]-3-(Butoxycarbonyl)amino-2-(triisopropylsilyloxy) benzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 19a werden 20 mg (28 *µ* mol) der nach Beispiel 25a dargestellten Verbindung umgesetzt. Nach Aufarbeitung und Reinigung isoliert man 7 mg (10 µmol, 36%) der Titelverbindung als farbloses Öl.
¹H-NMR (CDCl₃): δ = 0,68-1,61 (42H), 2,03 (1H), 2,16 (1H), 2,59 (1H), 3,05 (1H), 3,83 (1H), 4,05 (1H), 4,66 (1H), 4,87 (1H), 5,27 (1H), 6,23 (1H), 7,07-7,50 (9H) ppm.

### BEISPIEL 37

### [1R-[1α,2β(2R*,3S*),4α,4aβ,9β,10aβ]]-3-[(1-Oxobutyl)amino]-2-hydroxybenzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 24 mg (36 *µ* mol) der nach Beispiel 37a dargestellten Verbindung umgesetzt. Nach Aufarbeitung und Reinigung isoliert man 13 mg (25 *µ* mol, 70%) der Titelverbindung als farbloses Öl.
¹H-NMR (CDCl₃): δ = 0,78 (3H), 0,82 (3H), 0,90 (3H), 0,98 (3H), 1,35 (3H), 1,62 (2H), 1,81 (1H), 2,10 (2H), 2,23 (2H), 2,32 (1H), 2,69 (1H), 3,08 (1H), 3,30 (1H), 4,54 (1H), 5,07 (1H), 5,49 (111), 7,03 (1H), 7,12-7,33 (6H), 7,39 (2H), 7,48 (1H) ppm.

### BEISPIEL 37a

### [1R-[1α,2β(2R*,3S*),4α,4aβ,9β,10aβ]]-3-[(1-Oxobutyl)amino]-2-(triisopropylsilyloxy) benzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 19a werden 60 mg (89 *µ* mol) der nach Beispiel 13a dargestellten Verbindung umgesetzt. Nach Aufarbeitung und Reinigung isoliert man 24 mg (36 *µ* mol, 40%) der Titelverbindung als farbloses Öl.
¹H-NMR (CDCl₃): δ = 0,75 (3H), 0,78-1,18 (30H), 1,38 (3H), 1,57-1,74 (3H), 2,08 (2H), 2,22 (1H), 2,28 (3H), 2,62 (1H), 3,05 (1H), 4,63 (1H), 4,96 (1H), 5,51 (1H), 6,98 (1H), 7,09-7,40 (8H), 7,46 (1H) ppm.

### BEISPIEL 38

### [1R-[1α,2β(2R*,3S*),4α,4aβ,9β,10aβ]]-3-[3-Cyclopentyl-1-oxopropyl)amino]-2-hydroxybenzenpropansäure-9-methyl-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,9β,10aβ]]-3-[3-Cyclopentyl-1-oxopropyl)amino]-2 hydroxybenzenpropansäure-9-methyl-1,2,3,4,4a,9,10,10a-octahydro-1,12,12-trimethyl-4a,10a-epoxy-1,4-methanophenanthren-2-ylester (B)

In Analogie zu Beispiel 1a werden 10 mg (14 µmol) des nach Beispiel 38a dargestellten Gemisches umgesetzt. Nach Aufarbeitung und Reinigung isoliert man 2 mg (3,5 µmol, 25%) der Titelverbindung A sowie 2,4 mg (4,2 µmol, 30%) der Titelverbindung B jeweils als farbloses Öl.
¹H-NMR (CDCl₃) von A: δ = 0,79 (3H), 0,83-1,04 (8H), 1,30-1,70 (12H), 1,83 (1H), 2,09 (2H), 2,27 (2H), 2,35 (1H), 2,70 (1H), 3,08 (1H), 3,29 (1H), 4,56 (1H), 5,09 (1H), 5,45 (1H), 7,10-7,43 (9H), 7,48 (1H) ppm.
¹H-NMR (CDCl₃) von B: δ = 0,77 (3H), 0,84-1,07 (8H), 1,32 (3H), 1,38-1,70 (9H), 1,81 (1H), 1,98 (1H), 2,08 (1H), 2,24 (2H), 2,33 (1H), 2,68 (1H), 3,03 (1H), 3,16 (1H), 4,53 (1H), 5,17 (1H), 5,66 (1H), 6,96 (1H), 7,10-7,48 (9H) ppm.

### BEISPIEL 38a

### [1R-[1α,2β(2R*,3S*),4α,4aβ,9β,10aβ]]-3-[3-Cyclopentyl-1-oxopropyl)amino]-2-(triisopropylsilyloxy) benzenpropansäure-9-methyl-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,9β,10aβ]]-3-[3-Cyclopentyl-1-oxopropyl)amino]-2-(triisopropylsilyloxy) benzenpropansäure-9-methyl-1,2,3,4,4a,9,10,10a-octahydro-1,12,12-trimethyl-4a,10a-epoxy-1,4-methanophenanthren-2-ylester (B)

In Analogie zu Beispiel 19a werden 18 mg (25 *µ* mol) des nach Beispiel 20a dargestellten Gemisches umgesetzt. Nach Aufarbeitung und Reinigung isoliert man 10 mg (14 *µ* mol, 56%) der Titelverbindungen als farbloses Öl.

### BEISPIEL 39

### [1R-[1α,2β(2R*,3S*),4α,4aβ,9β,10aβ]]-3-[[[(1-Methyl)ethylamino]carbonyl] amino]-2-hydroxybenzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 4 mg (6 µmol) der nach Beispiel 39a dargestellten Verbindung umgesetzt. Nach Aufarbeitung und Reinigung isoliert man 2 mg (3,8 µmol, 63%) der Titelverbindung als farbloses Öl.
¹H-NMR (CDCl₃): δ = 0,65 (3H), 0,71 (3H), 0,84 (3H), 0,95 (3H), 1,07 (3H), 1,40 (3H), 1,83 (1H), 2,19 (2H), 2,46 (1H), 2,75 (1H), 2,93 (1H), 3,13 (1H), 3,67 (1H), 4,58 (1H), 4,77 (1H), 5,08 (1H), 5,13 (1H), 6,20 (1H), 7,14-7,36 (8H), 7,49 (1H) ppm.

### BEISPIEL 39a

### [1R-[1α,2β(2R*,3S*),4α,4aβ,9β,10aβ]]-3-[[[(1-Methyl)ethylamino]carbonyl] amino]-2-(triisopropylsilyloxy) benzenpropansäure-9-methyl-1,2,3,4,4a,9,10,10a-octahydro-1,12,12-trimethyl-4a,10a-epoxy-1,4-methanophenanthren-2-ylester

In Analogie zu Beispiel 19a werden 10 mg (15 *µ* mol) der nach Beispiel 29a dargestellten Verbindung umgesetzt. Nach Aufarbeitung und Reinigung isoliert man 4 mg (6 µmol, 40%) der Titelverbindung als farbloses Öl.
¹H-NMR (CDCl₃): δ = 0,65 (6H), 0,75-1,15 (30H), 1,39 (3H), 1,72 (1H), 2,18 (2H), 2,38 (1H), 2,70 (1H), 3,11 (1H), 3,68 (1H), 4,68 (2H), 4,97 (1H), 5,20 (1H), 6,04 (1H), 7,12-7,36 (8H), 7,47 (1H) ppm.

### BEISPIEL 40

### [1R-[1α,2β(2R*,3S*),4α,4aβ,10aβ]]-3-[(1-Oxobutyl)methylamino]-2-hydroxybenzenpropansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 9 mg (13 µmol) der nach Beispiel 40a dargestellten Verbindung umgesetzt. Nach Aufarbeitung und Reinigung isoliert man 5,6 mg (10,6 µmol, 81%) der Titelverbindung als farbloses Öl.
¹H-NMR (CDCl₃): δ = 0,80 (3H), 0,87-1,06 (9H), 1,53-1,83 (3H), 2,22-2,40 (3H), 2,65 (1H), 2,70 (1H), 2,91 (3H), 3,02 (1H), 4,83 (2H), 5,02 (1H), 5,05 (1H), 5,46 (1H), 5,72 (1H), 7,21-7,40 (7H), 7,45 (1H), 7,50 (1H) ppm.

### BEISPIEL 40a

### [1R-[1α,2β(2R*,3S*),4α,4aβ,10aβ]]-3-[(1-Oxobutyl)methylamino]-2-(triisopropylsilyloxy)benzenpropansäure-9-methylen-1,2,3,4,4a,9,10,10a-octahydro-1,12,12-trimethyl-4a,10a-epoxy-1,4-methanophenanthren-2-ylester

Zu 1 mg einer 60%igen öligen Natriumhydrid-Dispersion gibt man unter einer Atmosphäre aus trockenem Argon die Lösung von 15 mg (22 µmol) der nach Beispiel 13a dargestellten Verbindung in 0,3 ml wasserfreiem Tetrahydrofuran, versetzt mit 2,1 µl Iodmethan und rührt 2 Stunden bei 23°C. Nach Aufarbeitung isoliert man 9 mg (13 µmol, 60%) der Titelverbindung als farbloses Öl, das man direkt weiter umsetzt.

### BEISPIEL 41

### [1R-[1α,2β(2R*,3S*),4α,4aβ,10aβ]]-3-[(1-Oxobutyl)amino]-2-acetoxybenzenpropansäure-9-methylen-1,2,3,4,4a,9,10,10a-octahydro-1,12,12-trimethyl-4a,10a-epoxy-1,4-methanophenanthren-2-ylester

Die Lösung von 11 mg (21 µmol) der nach Beispiel 13 dargestellten Verbindung in 0,5 ml wasserfreiem Pyridin versetzt man mit 19 µl Acetanhydrid und rührt bei 23°C 1 Stunde unter einer Atmosphäre aus trockenem Argon. Man gießt in eine gesättigte Natriumhydrogencarbonatlösung, extrahiert mit Dichlormethan, wäscht die organische Phase mit gesättigter Natriumchloridlösung und trocknet über Magnesiumsulfat. Den nach Filtration und Lösungsmittelabzug erhaltenen Rückstand reinigt man durch Chromatographie an einer analytischen Dünnschichtplatte. Als Laufmittel dient ein Gemisch aus n-Hexan und Ethylacetat, als Elutionsmittel ein Gemisch aus Diethylether und 2-Propanol. Isoliert werden 8 mg (14 µmol, 68%) der Titelverbindung als farbloses Öl.
¹H-NMR (CDCl₃): δ = 0,80 (3H), 0,83 (3H), 0,89 (3H), 1,07 (3H), 1,48 (1H), 1,60 (2H), 2,10-2,30 (3H), 2,20 (3H), 2,68 (1H), 2,81 (1H), 3,07 (1H), 4,97 (1H), 5,02 (1H), 5,32 (1H), 5,52 (1H), 5,69 (1H), 7,20-7,64 (10H) ppm.

## Patentansprüche

1. Bomeolderivate der allgemeinen Formel I 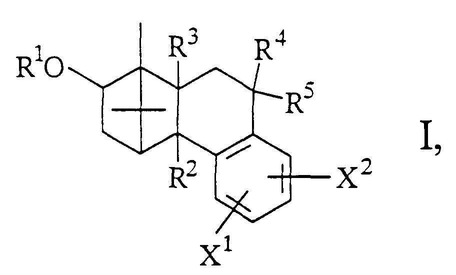 worin
R¹ C(O)-CH(OR⁶)-CH(NR^{7a}R^{7b})-R⁸, C(O)-CH(OR^{6a})-CH[NH(C(O)-CH(OR^{6b})-CH(NR^{7a}R^{7b})R⁸)]-R⁸,
R² Wasserstoff, -OH, C₁-C₁₀-Alkyl, C₁-C₁₀-Alkoxy, -OC(O)R^{9a}, -OSO₂R^{9a}, -OP(O)(OH)₂, NHR^{9a}, NR^{9a}R^{9b},
R³ Wasserstoff, -OH, C₁-C₁₀-Alkoxy, -OC(O)R^{9b}, -OSO₂R^{9b}, -OP(O)(OH)₂, oder
R², R³ gemeinsam ein Sauerstoffatom,
R⁴ Wasserstoff, C₁-C₁₀-Alkyl, -(CH₂)ₙ-OR^{11a},
R⁵ Wasserstoff, C₁-C₁₀-Alkyl, -(CH₂)ₚ-OR^{11b}, oder
R⁴, R⁵ gemeinsam ein Sauerstoffatom, eine =CHR¹⁰-Gruppe,
R^{6a}, R^{6b} gleich oder verschieden sind und R⁶ bedeuten,
R^{7a}, R^{7b} gleich oder verschieden sind und R⁷ bedeuten,
n 0 bis 8
p 1 bis 8
R⁷ -C(O)R¹², -SO₂R¹², -C(O)OR¹², -C(O)SR¹², -C(O)NHR^{9d}, -C(O)NR^{9d}R^{9e}, C₁-C₁₀-Alkyl, 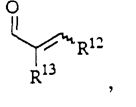
R⁸ Phenyl,
R^{9a-e}, R¹² gleich oder verschieden sind und C₁-C₁₀-Alkyl, C₄-C₈-Cycloalkyl, Aryl, C₇-C₁₆-Aralkyl bedeuten,
R¹⁰ Wasserstoff, C₁-C₁₀-Alkyl, -(CH₂)ₛ-OR¹⁴,
s 1 bis 8
R⁶, R^{11a,b}, R¹⁴ gleich oder verschieden sind und Wasserstoff, C₁-C₁₀-Alkyl, Aryl, C₁-C₁₀-Acyl, C₇-C₁₆-Aralkyl, -SO₂R^{9c}, -P(O)(OH)₂ bedeuten,
R¹³, R^{15a,b} gleich oder verschieden sind und Wasserstoff, C₁-C₁₀-Alkyl, Aryl, C₇-C₁₆-Aralkyl bedeuten,
X¹, X² gleich oder verschieden sind und X bedeuten,
X Wasserstoff, Halogen, -OH, -NO₂, -N₃, -CN, -NR^{15a}R^{15b}, -NHSO₂R^{15a}, -CO₂R¹⁵, C₁-C₁₀-Alkyl, C₁-C₁₀-Alkoxy, C₁-C₁₀-Acyloxy, C₁-C₁₀-Acyl sein kann
und, falls R¹⁵ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

2. Arzneimittel, bestehend aus einer oder mehrerer Verbindungen des Anspruches 1 und üblicher Hilfs-, Träger- und Zusatzstoffe.

3. Verfahren zur Herstellung von Borneolderivaten der allgemeinen Formel I gemäß Patentanspruch 1, das **dadurch gekennzeichnet, daß** man ein Olefin der allgemeinen Formel II 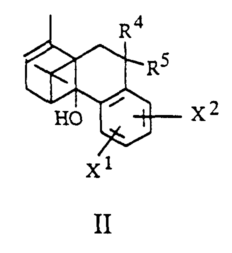 in dem R⁴, R⁵, X¹ und X² die oben genannten Bedeutungen haben und in X¹ oder X² enthaltene Hydroxylgruppen gegebenfalls geschützt sind, epoxidiert und das gebildete Epoxid ohne Isolation zu einem Alkohol der allgemeinen Formel III 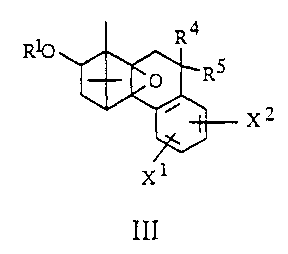 in dem R⁴, R⁵, X¹ und X² die oben genannten Bedeutungen haben und in R¹, X¹ oder X² enthaltene Hydroxylgruppen gegebenfalls geschützt sind, umlagert und dieses Umlagerungsprodukt in ein Derivat der allgemeinen Formel I überführt.

## Claims

1. Borneol derivatives of the general formula I 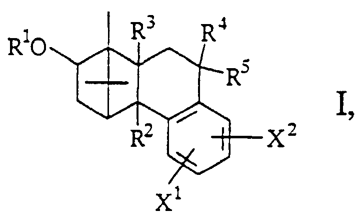 in which
R¹ denotes C(O)-CH(OR⁶)-CH(NR^{7a}R^{7b})-R⁸, C(O)-CH(OR^{6a})-CH[NH(C(O)-CH(OR^{6b})-CH(NR^{7a}R^{7b})-R⁸)]-R⁸,
R² denotes hydrogen, -OH, C₁-C₁₀-alkyl, C₁-C₁₀-alkoxy, -OC(O)R^{9a}, -OSO₂R^{9a}, -OP(O)(OH)₂, NHR^{9a}, NR^{9a}R^{9b},
R³ denotes hydrogen, -OH, C₁-C₁₀-alkoxy, -OC(O)R^{9b}, -OSO₂R^{9b}, -OP(O)(OH)₂ or
R² and R³ together denote an oxygen atom,
R⁴ denotes hydrogen, C₁-C₁₀-alkyl, -(CH₂)ₙ-OR^{11a},
R⁵ denotes hydrogen, C₁-C₁₀-alkyl, -(CH₂)ₚ-OR^{11b}, or
R⁴ and R⁵ together denote an oxygen atom, a =CHR¹⁰ group,
R^{6a} and R^{6b} are identical or different and denote R⁶,
R^{7a} and R^{7b} are identical or different and denote R⁷,
n is from 0 to 8,
p is from 1 to 8,
R⁷ denotes -C(O)R¹², -SO₂R¹², -C(O)OR¹², -C(O)SR¹², -C(O)NHR^{9d}, -C(O)NR^{9d}R^{9e}, C₁-C₁₀-alkyl, 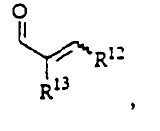
R⁸ denotes phenyl,
R^{9a-e} and R¹² are identical or different and denote C₁-C₁₀-alkyl, C₄-C₈-cycloalkyl, aryl, C₇-C₁₆-aralkyl,
R¹⁰ denotes hydrogen, C₁-C₁₀-alkyl, - (CH₂)ₛ-OR¹⁴,
s is from 1 to 8,
R⁶, R^{11a,b} and R¹⁴ are identical or different and denote hydrogen, C₁-C₁₀-alkyl, aryl, C₁-C₁₀-acyl, C₇-C₁₆-aralkyl, -SO₂R^{9c}, -P(O)(OH)₂,
R¹³ and R^{15a,b} are identical or different and denote hydrogen, C₁-C₁₀-alkyl, aryl, C₇-C₁₆-aralkyl,
X¹ and X² are identical or different and denote X,
X can be hydrogen, halogen, -OH, -NO₂, -N₃, -CN, -NR^{15a}R^{15b}, -NHSO₂R^{15a}, -CO₂R¹⁵, C₁-C₁₀-alkyl, C₁-C₁₀-alkoxy, C₁-C₁₀-acyloxy, C₁-C₁₀-acyl
and
if R¹⁵ is hydrogen, their salts with physiologically acceptable bases, and their α-, β- or γ-cyclodextrin clathrates, and the liposome-encapsulated compounds of the formula I.

2. Pharmaceutical, comprising one or more compounds of Claim 1 and customary auxiliaries, carriers and additives.

3. Process for preparing borneol derivatives of the general formula I according to Patent Claim 1, **characterized in that** an olefin of the general formula II 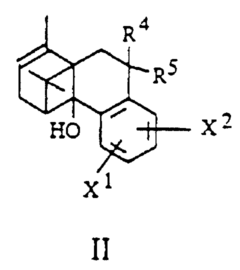 in which R⁴, R⁵, X¹ and X² are as defined above and any hydroxyl groups present in X¹ or X² are optionally protected, is epoxidized and the resulting epoxide is rearranged without isolation into an alcohol of the general formula III 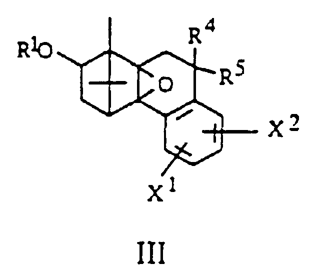 in which R⁴, R⁵, X¹ and X² are as defined above and any hydroxyl groups present in R¹, X¹ or X² are optionally protected, and this rearrangement product is converted into a derivative of the general formula I.

## Revendications

1. Dérivés du bornéol de formule générale I 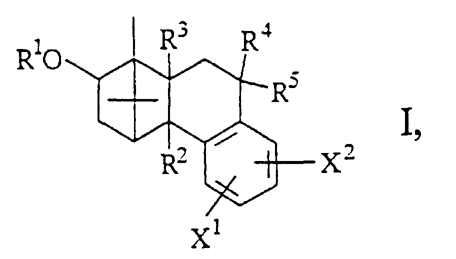 dans laquelle
R¹ signifie C(O)-CH(OR⁶)-CH(NR^{7a}R^{7b})-R⁸, C(O)-CH(OR^{6a})-CH[NH(C(O)-CH(OR^{6b})-CH(NR^{7a}R^{7b})-R⁸)]-R⁸,
R² signifie hydrogène, -OH, alkyle en C₁-C₁₀, alkoxy en C₁-C₁₀, -OC(O)R^{9a}, -OSO₂R^{9a}, -OP(O)(OH)₂, NHR^{9a}, NR^{9a}R^{9b},
R³ signifie hydrogène, -OH, alkoxy en C₁-C₁₀,-OC(O)R^{9b}, -OSO₂R^{9b}, -OP(O)(OH)₂, ou
R², R³ signifient ensemble un atome d'oxygène,
R⁴ signifie hydrogène, alkyle en C₁-C₁₀, (CH₂)ₙ-OR^{11a},
R⁵ signifie hydrogène, alkyle en C₁-C₁₀, (CH₂)ₚ-OR^{11b}, ou
R⁴, R⁵ signifient ensemble un atome d'oxygène, un groupement =CHR¹⁰,
R^{6a}, R^{6b} sont identiques ou différents et signifient R⁶,
R^{7a}, R^{7b} sont identiques ou différents et signifient R⁷,
n signifie 0 à 8
p signifie 1 à 8
R⁷ signifie -C(O)R¹², -SO₂R¹², -C(O)OR¹², -C(O)SR¹²,-C(O)NHR^{9d}, -C(O)NR^{9d}R^{9e},
alkyle en C₁-C₁₀, 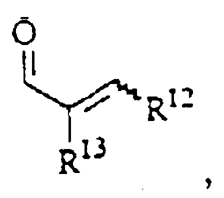
R⁸ signifie phényle,
R^{9a-e}, R¹² sont identiques ou différents et signifient alkyle en C₁-C₁₀, cycloalkyle en C₄-C₈, aryle, aralkyle en C₇-C₁₆,
R¹⁰ signifie hydrogène, alkyle en C₁-C₁₀, -(CH₂)ₛ-OR¹⁴,
s signifie 1 à 8
R⁶, R^{11a,b}, R¹⁴ sont identiques ou différents et signifient hydrogène, alkyle en C₁-C₁₀, aryle, acyle en C₁-C₁₀, aralkyle en C₇-C₁₆, -SO₂R^{9c}, -P(O) (OH)₂,
R¹³, R^{15a,b} sont identiques ou différents et signifient hydrogène, alkyle en C₁-C₁₀, aryle, aralkyle en C₇-C₁₆,
X¹, X² sont identiques ou différents et signifient X,
X peut être hydrogène, halogène, -OH, -NO₂, -N₃, -CN, -NR^{15a}R^{15b}, -NHSO₂R^{15a}, -CO₂R¹⁵, alkyle en C₁-C₁₀, alkoxy en C₁-C₁₀, acyloxy en C₁-C₁₀, acyle en C₁-C₁₀,
et, si R¹⁵ signifie un hydrogène, leurs sels avec des bases physiologiquement acceptables, ainsi que les clathrates de α-cyclodextrine, de β-cyclodextrine ou de γ-cyclodextrine, ainsi que les composés de formule I encapsulés par des liposomes.

2. Médicament, constitué d'un ou de plusieurs composés de la revendication 1 et d'adjuvants, de substances support et d'additifs usuels.

3. Procédé pour la préparation de dérivés du bornéol de formule générale I selon la revendication 1, **caractérisé en ce qu'**on époxyde une oléfine de formule générale II 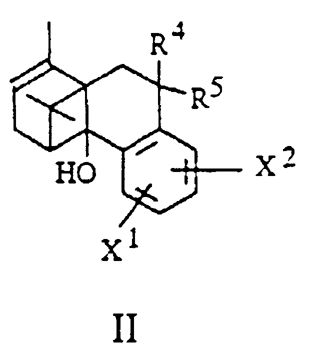 dans laquelle R⁴, R⁵, X¹ et X² ont les significations susmentionnées et les groupements hydroxyle contenus dans X¹ ou X² sont le cas échéant protégés et en ce qu'on réarrange l'époxyde formé sans isolement en un alcool de formule générale III 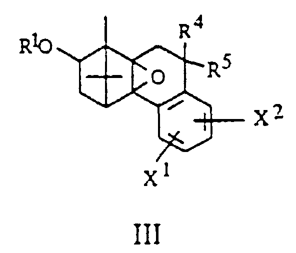 dans laquelle R⁴, R⁵, X¹ et X² ont les significations susmentionnées et les groupements hydroxyle contenus dans R¹, X¹ ou X² sont le cas échéant protégés et en ce qu'on transforme ce produit de réarrangement en un dérivé de formule générale I.
